(19) Europäisches Patentamt
European Patent Office
Office européen des brevets

(11) EP 4 062 936 A1

(12) EUROPEAN PATENT APPLICATION

(43) Date of publication:
**28.09.2022 Bulletin 2022/39**

(21) Application number: **22155572.5**

(22) Date of filing: **14.08.2012**

(51) International Patent Classification (IPC):
***A61K 39/395*** $^{(2006.01)}$ ***C07K 16/28*** $^{(2006.01)}$
***A61K 31/4184*** $^{(2006.01)}$ ***A61P 35/00*** $^{(2006.01)}$
***A61P 35/02*** $^{(2006.01)}$ ***A61P 43/00*** $^{(2006.01)}$
***C07K 16/30*** $^{(2006.01)}$

(52) Cooperative Patent Classification (CPC):
(C-Sets available)
**A61K 39/3955; A61K 31/4184; A61K 39/39558; A61P 35/00; A61P 35/02; A61P 43/00; C07K 16/2803; C07K 16/30;** A61K 2039/505

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.08.2011 EP 11177658**
**16.08.2011 US 201161523861 P**
**16.05.2012 US 201261647539 P**
**01.06.2012 US 201261654097 P**

(62) Document number(s) of the earlier application(s) in accordance with Art. 76 EPC:
**12747923.6 / 2 744 515**

(71) Applicant: **MorphoSys AG**
**82152 Planegg (DE)**

(72) Inventors:
- **AMERSDORFER, Jutta**
**85421 Hebertshausen (DE)**
- **STEIDL, Stefan**
**81241 München (DE)**
- **WINDERLICH, Mark**
**81371 München (DE)**
- **KROHN, Susanne**
**81241 München (DE)**
- **ROJKJAER, Lisa**
**CH-8908 Hedingen (CH)**

(74) Representative: **Carpmaels & Ransford LLP**
**One Southampton Row**
**London WC1B 5HA (GB)**

Remarks:
- A request for correction of the drawings, drawings and sequence listing has been filed pursuant to Rule 139 EPC. A decision on the request will be taken during the proceedings before the Examining Division (Guidelines for Examination in the EPO, A-V, 3.).
- This application was filed on 08-02-2022 as a divisional application to the application mentioned under INID code 62.
- Claims filed after the date of filing of the application / after the date of receipt of the divisional application (Rule 68(4) EPC).

# (54) COMBINATION THERAPY WITH AN ANTI-CD19 ANTIBODY AND A NITROGEN MUSTARD

(57) The present disclosure describes a pharmaceutical combination of an anti-CD19 antibody and a nitrogen mustard for the treatment of non-Hodgkin's lymphoma, chronic lymphocytic leukemia and/or acute lymphoblastic leukemia.

EP 4 062 936 A1

**Figure 5**

Normalized specific killing; SD; MEC-1 target cells pretreated with Bendamustine (Ben) for 48 h before ADCC; pool of 3 independent experiments with 3 different effector cell donors

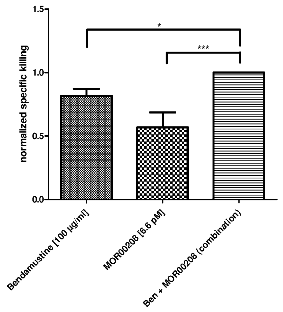


The figure shows the averages from the data shown in Table 2.

(52) Cooperative Patent Classification (CPC): (Cont.)

C-Sets
**A61K 31/4184, A61K 2300/00;**
**A61K 39/3955, A61K 2300/00;**
**A61K 39/39558, A61K 2300/00**

SEQUENCE LISTING

<110> MorphoSys AG

<120> Combinations and uses thereof

<150> EP11177658.9

<151> August 16, 2011

<150> US 61/523,861

<151> August 16, 2011

<150> US 61/647,539

<151> May 16, 2012

<150> US 61/654,097

<151> June 1, 2012

<160> 13

<170> BiSSAP 1.0

<210> 1
<211> 5
<212> PRT
<213> synthetic construct

<220>
<221> SOURCE
<222> 1..5
<223> /mol_type="protein"
/note="HCDR1 "
/organism="synthetic construct"

<400> 1
Ser Tyr Val Met His
1 5

<210> 2
<211> 6
<212> PRT
<213> synthetic construct

<220>
<221> SOURCE
<222> 1..6
<223> /mol_type="protein"
/note="HCDR2 "
/organism="synthetic construct"

<400> 2
Asn Pro Tyr Asn Asp Gly
1 5

<210> 3
<211> 12
<212> PRT
<213> synthetic construct

<220>
<221> SOURCE
<222> 1..12
<223> /mol_type="protein"
/note="HCDR3 "
/organism="synthetic construct"

<400> 3
Gly Thr Tyr Tyr Tyr Gly Thr Arg Val Phe Asp Tyr
1 5 10

<210> 4
<211> 16
<212> PRT
<213> synthetic construct

<220>
<221> SOURCE
<222> 1..16
<223> /mol_type="protein"
/note="LCDR1 "
/organism="synthetic construct"

<400> 4
Arg Ser Ser Lys Ser Leu Gln Asn Val Asn Gly Asn Thr Tyr Leu Tyr
1 5 10 15

<210> 5
<211> 7
<212> PRT
<213> synthetic construct

<220>
<221> SOURCE
<222> 1..7
<223> /mol_type="protein"
/note="LCDR2 "
/organism="synthetic construct"

<400> 5
Arg Met Ser Asn Leu Asn Ser
1 5

<210> 6
<211> 9
<212> PRT
<213> synthetic construct

<220>
<221> SOURCE
<222> 1..9
<223> /mol_type="protein"
/note="LCDR3 "
/organism="synthetic construct"

<400> 6
Met Gln His Leu Glu Tyr Pro Ile Thr
1 5

<210> 7
<211> 556
<212> PRT
<213> Homo sapiens

```
<220>
<221> SOURCE
<222> 1..556
<223> /mol_type="protein"
      /note="CD19"
      /organism="Homo sapiens"

<400> 7
Met Pro Pro Pro Arg Leu Leu Phe Phe Leu Leu Phe Leu Thr Pro Met
1               5                   10                  15
Glu Val Arg Pro Glu Glu Pro Leu Val Val Lys Val Glu Glu Gly Asp
            20                  25                  30
Asn Ala Val Leu Gln Cys Leu Lys Gly Thr Ser Asp Gly Pro Thr Gln
        35                  40                  45
Gln Leu Thr Trp Ser Arg Glu Ser Pro Leu Lys Pro Phe Leu Lys Leu
    50                  55                  60
Ser Leu Gly Leu Pro Gly Leu Gly Ile His Met Arg Pro Leu Ala Ile
65                  70                  75                  80
Trp Leu Phe Ile Phe Asn Val Ser Gln Gln Met Gly Gly Phe Tyr Leu
                85                  90                  95
Cys Gln Pro Gly Pro Pro Ser Glu Lys Ala Trp Gln Pro Gly Trp Thr
            100                 105                 110
Val Asn Val Glu Gly Ser Gly Glu Leu Phe Arg Trp Asn Val Ser Asp
        115                 120                 125
Leu Gly Gly Leu Gly Cys Gly Leu Lys Asn Arg Ser Ser Glu Gly Pro
    130                 135                 140
Ser Ser Pro Ser Gly Lys Leu Met Ser Pro Lys Leu Tyr Val Trp Ala
145                 150                 155                 160
Lys Asp Arg Pro Glu Ile Trp Glu Gly Glu Pro Pro Cys Leu Pro Pro
                165                 170                 175
Arg Asp Ser Leu Asn Gln Ser Leu Ser Gln Asp Leu Thr Met Ala Pro
            180                 185                 190
Gly Ser Thr Leu Trp Leu Ser Cys Gly Val Pro Pro Asp Ser Val Ser
        195                 200                 205
Arg Gly Pro Leu Ser Trp Thr His Val His Pro Lys Gly Pro Lys Ser
    210                 215                 220
Leu Leu Ser Leu Glu Leu Lys Asp Asp Arg Pro Ala Arg Asp Met Trp
225                 230                 235                 240
Val Met Glu Thr Gly Leu Leu Leu Pro Arg Ala Thr Ala Gln Asp Ala
                245                 250                 255
Gly Lys Tyr Tyr Cys His Arg Gly Asn Leu Thr Met Ser Phe His Leu
            260                 265                 270
Glu Ile Thr Ala Arg Pro Val Leu Trp His Trp Leu Leu Arg Thr Gly
        275                 280                 285
Gly Trp Lys Val Ser Ala Val Thr Leu Ala Tyr Leu Ile Phe Cys Leu
    290                 295                 300
Cys Ser Leu Val Gly Ile Leu His Leu Gln Arg Ala Leu Val Leu Arg
305                 310                 315                 320
Arg Lys Arg Lys Arg Met Thr Asp Pro Thr Arg Arg Phe Phe Lys Val
                325                 330                 335
Thr Pro Pro Pro Gly Ser Gly Pro Gln Asn Gln Tyr Gly Asn Val Leu
            340                 345                 350
Ser Leu Pro Thr Pro Thr Ser Gly Leu Gly Arg Ala Gln Arg Trp Ala
        355                 360                 365
Ala Gly Leu Gly Gly Thr Ala Pro Ser Tyr Gly Asn Pro Ser Ser Asp
    370                 375                 380
Val Gln Ala Asp Gly Ala Leu Gly Ser Arg Ser Pro Pro Gly Val Gly
385                 390                 395                 400
Pro Glu Glu Glu Glu Gly Glu Gly Tyr Glu Glu Pro Asp Ser Glu Glu
                405                 410                 415
Asp Ser Glu Phe Tyr Glu Asn Asp Ser Asn Leu Gly Gln Asp Gln Leu
            420                 425                 430
```

```
Ser Gln Asp Gly Ser Gly Tyr Glu Asn Pro Glu Asp Glu Pro Leu Gly
        435                 440                 445
Pro Glu Asp Glu Asp Ser Phe Ser Asn Ala Glu Ser Tyr Glu Asn Glu
    450                 455                 460
Asp Glu Glu Leu Thr Gln Pro Val Ala Arg Thr Met Asp Phe Leu Ser
465                 470                 475                 480
Pro His Gly Ser Ala Trp Asp Pro Ser Arg Glu Ala Thr Ser Leu Gly
                485                 490                 495
Ser Gln Ser Tyr Glu Asp Met Arg Gly Ile Leu Tyr Ala Ala Pro Gln
            500                 505                 510
Leu Arg Ser Ile Arg Gly Gln Pro Gly Pro Asn His Glu Glu Asp Ala
        515                 520                 525
Asp Ser Tyr Glu Asn Met Asp Asn Pro Asp Gly Pro Asp Pro Ala Trp
    530                 535                 540
Gly Gly Gly Gly Arg Met Gly Thr Trp Ser Thr Arg
545                 550                 555

<210> 8
<211> 450
<212> PRT
<213> synthetic construct

<220>
<221> SOURCE
<222> 1..450
<223> /mol_type="protein"
      /note="Heavy chain RefMab33"
      /organism="synthetic construct"

<400> 8
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ala
            20                  25                  30
Gly Met Ser Val Gly Trp Ile Arg Gln Pro Pro Gly Lys Ala Leu Glu
        35                  40                  45
Trp Leu Ala Asp Ile Trp Trp Asp Asp Lys Lys His Tyr Asn Pro Ser
    50                  55                  60
Leu Lys Asp Arg Leu Thr Ile Ser Lys Asp Thr Ser Lys Asn Gln Val
65                  70                  75                  80
Val Leu Lys Val Thr Asn Met Asp Pro Ala Asp Thr Ala Thr Tyr Tyr
                85                  90                  95
Cys Ala Arg Asp Met Ile Phe Asn Phe Tyr Phe Asp Val Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Asp Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
```

```
            260                 265                 270
Asp Pro Glu Val Gln Phe Asn Trp Tyr Val Asp Gly Val Glu Val His
        275                 280                 285
Asn Ala Lys Thr Lys Pro Arg Glu Glu Gln Phe Asn Ser Thr Phe Arg
    290                 295                 300
Val Val Ser Val Leu Thr Val Val His Gln Asp Trp Leu Asn Gly Lys
305                 310                 315                 320
Glu Tyr Lys Cys Lys Val Ser Asn Lys Ala Leu Pro Ala Pro Glu Glu
                325                 330                 335
Lys Thr Ile Ser Lys Thr Lys Gly Gln Pro Arg Glu Pro Gln Val Tyr
            340                 345                 350
Thr Leu Pro Pro Ser Arg Glu Glu Met Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Met
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450
```

<210> 9
<211> 213
<212> PRT
<213> synthetic construct

<220>
<221> SOURCE
<222> 1..213
<223> /mol_type="protein"
/note="Light chain RefMab33"
/organism="synthetic construct"

<400> 9

```
Asp Ile Gln Met Thr Gln Ser Pro Ser Thr Leu Ser Ala Ser Val Gly
1               5                   10                  15
Asp Arg Val Thr Ile Thr Cys Ser Ala Ser Ser Arg Val Gly Tyr Met
            20                  25                  30
His Trp Tyr Gln Gln Lys Pro Gly Lys Ala Pro Lys Leu Leu Ile Tyr
        35                  40                  45
Asp Thr Ser Lys Leu Ala Ser Gly Val Pro Ser Arg Phe Ser Gly Ser
    50                  55                  60
Gly Ser Gly Thr Glu Phe Thr Leu Thr Ile Ser Ser Leu Gln Pro Asp
65                  70                  75                  80
Asp Phe Ala Thr Tyr Tyr Cys Phe Gln Gly Ser Gly Tyr Pro Phe Thr
                85                  90                  95
Phe Gly Gly Gly Thr Lys Val Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
```

```
Cys Glu Val Thr His Gln Gly Leu Ser Ser Pro Val Thr Lys Ser Phe
        195                 200                 205
Asn Arg Gly Glu Cys
    210

<210> 10
<211> 121
<212> PRT
<213> synthetic construct

<220>
<221> SOURCE
<222> 1..121
<223> /mol_type="protein"
      /note="variable heavy chain"
      /organism="synthetic construct"

<400> 10
Glu Val Gln Leu Val Glu Ser Gly Gly Gly Leu Val Lys Pro Gly Gly
1               5                   10                  15
Ser Leu Lys Leu Ser Cys Ala Ala Ser Gly Tyr Thr Phe Thr Ser Tyr
            20                  25                  30
Val Met His Trp Val Arg Gln Ala Pro Gly Lys Gly Leu Glu Trp Ile
        35                  40                  45
Gly Tyr Ile Asn Pro Tyr Asn Asp Gly Thr Lys Tyr Asn Glu Lys Phe
    50                  55                  60
Gln Gly Arg Val Thr Ile Ser Ser Asp Lys Ser Ile Ser Thr Ala Tyr
65                  70                  75                  80
Met Glu Leu Ser Ser Leu Arg Ser Glu Asp Thr Ala Met Tyr Tyr Cys
                85                  90                  95
Ala Arg Gly Thr Tyr Tyr Tyr Gly Thr Arg Val Phe Asp Tyr Trp Gly
            100                 105                 110
Gln Gly Thr Leu Val Thr Val Ser Ser
        115                 120

<210> 11
<211> 112
<212> PRT
<213> synthetic construct

<220>
<221> SOURCE
<222> 1..112
<223> /mol_type="protein"
      /note="variable light chain"
      /organism="synthetic construct"

<400> 11
Asp Ile Val Met Thr Gln Ser Pro Ala Thr Leu Ser Leu Ser Pro Gly
1               5                   10                  15
Glu Arg Ala Thr Leu Ser Cys Arg Ser Ser Lys Ser Leu Gln Asn Val
            20                  25                  30
Asn Gly Asn Thr Tyr Leu Tyr Trp Phe Gln Gln Lys Pro Gly Gln Ser
        35                  40                  45
Pro Gln Leu Leu Ile Tyr Arg Met Ser Asn Leu Asn Ser Gly Val Pro
    50                  55                  60
Asp Arg Phe Ser Gly Ser Gly Ser Gly Thr Glu Phe Thr Leu Thr Ile
65                  70                  75                  80
Ser Ser Leu Glu Pro Glu Asp Phe Ala Val Tyr Tyr Cys Met Gln His
                85                  90                  95
Leu Glu Tyr Pro Ile Thr Phe Gly Ala Gly Thr Lys Leu Glu Ile Lys
            100                 105                 110
```

```
<210> 12
<211> 329
<212> PRT
<213> synthetic construct

<220>
<221> SOURCE
<222> 1..329
<223> /mol_type="protein"
      /note="heavy chain constant domain"
      /organism="synthetic construct"

<400> 12
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Trp Thr Val Pro Ser Ser Ser Leu Gly Thr Gln Thr Tyr
65                  70                  75                  80
Ile Cys Asn Val Asn His Lys Pro Ser Asn Thr Lys Val Asp Lys Lys
                85                  90                  95
Val Glu Pro Lys Ser Cys Asp Lys Thr His Thr Cys Pro Pro Cys Pro
            100                 105                 110
Ala Pro Glu Leu Leu Gly Gly Pro Asp Val Phe Leu Phe Pro Pro Lys
        115                 120                 125
Pro Lys Asp Thr Leu Met Ile Ser Arg Thr Pro Glu Val Thr Cys Val
    130                 135                 140
Val Val Asp Val Ser His Glu Asp Pro Glu Val Gln Phe Asn Trp Tyr
145                 150                 155                 160
Val Asp Gly Val Glu Val His Asn Ala Lys Thr Lys Pro Arg Glu Glu
                165                 170                 175
Gln Phe Asn Ser Thr Phe Arg Val Val Ser Val Leu Thr Val Val His
            180                 185                 190
Gln Asp Trp Leu Asn Gly Lys Glu Tyr Lys Cys Lys Val Ser Asn Lys
        195                 200                 205
Ala Leu Pro Ala Pro Glu Glu Lys Thr Ile Ser Lys Thr Lys Gly Gln
    210                 215                 220
Pro Arg Glu Pro Gln Val Tyr Thr Leu Pro Pro Ser Arg Glu Glu Met
225                 230                 235                 240
Thr Lys Asn Gln Val Ser Leu Thr Cys Leu Val Lys Gly Phe Tyr Pro
                245                 250                 255
Ser Asp Ile Ala Val Glu Trp Glu Ser Asn Gly Gln Pro Glu Asn Asn
            260                 265                 270
Tyr Lys Thr Thr Pro Pro Met Leu Asp Ser Asp Gly Ser Phe Phe Leu
        275                 280                 285
Tyr Ser Lys Leu Thr Val Asp Lys Ser Arg Trp Gln Gln Gly Asn Val
    290                 295                 300
Phe Ser Cys Ser Val Met His Glu Ala Leu His Asn His Tyr Thr Gln
305                 310                 315                 320
Lys Ser Leu Ser Leu Ser Pro Gly Lys
                325

<210> 13
<211> 107
<212> PRT
<213> synthetic construct

<220>
<221> SOURCE
```

```
<222> 1..107
<223> /mol_type="protein"
      /note="light chain constant domain"
      /organism="synthetic construct"

<400> 13
Arg Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                85                  90                  95
Pro Val Thr Lys Ser Phe Asn Arg Gly Glu Cys
            100                 105
```

## Description

### Cross reference

This application claims the benefit of U.S. provisional application serial number 61/654,097 filed June 1, 2012, U.S. provisional application serial number 61/647,539 filed May 16, 2012, and U.S. provisional application serial number 61/523,861 filed August 16, 2011, which are incorporated by reference in their entireties.

### Field of the Invention

The present disclosure is related to a pharmaceutical combination of an anti-CD19 antibody and a nitrogen mustard for the treatment of non-Hodgkin's lymphoma, chronic lymphocytic leukemia and/or acute lymphoblastic leukemia.

### Background

B cells are lymphocytes that play a large role in the humoral immune response. They are produced in the bone marrow of most mammals, and represent 5-15% of the circulating lymphoid pool. The principal function of B cells is to make antibodies against various antigens, and are an essential component of the adaptive immune system.

Because of their critical role in regulating the immune system, disregulation of B cells is associated with a variety of disorders, such as lymphomas, and leukemias. These include non-Hodgkin's lymphoma (NHL), chronic lymphocytic leukemia (CLL) and acute lymphoblastic leukemia (ALL).

NHL is a heterogeneous malignancy originating from lymphocytes. In the United States (U.S.), the incidence is estimated at 65,000/year with mortality of approximately 20,000 (American Cancer Society, 2006; and SEER Cancer Statistics Review). The disease can occur in all ages, the usual onset begins in adults over 40 years, with the incidence increasing with age. NHL is characterized by a clonal proliferation of lymphocytes that accumulate in the lymph nodes, blood, bone marrow and spleen, although any major organ may be involved. The current classification system used by pathologists and clinicians is the World Health Organization (WHO) Classification of Tumours, which organizes NHL into precursor and mature B-cell or T-cell neoplasms. The PDQ is currently dividing NHL as indolent or aggressive for entry into clinical trials. The indolent NHL group is comprised primarily of follicular subtypes, small lymphocytic lymphoma, MALT (mucosa-associated lymphoid tissue), and marginal zone; indolent encompasses approximately 50% of newly diagnosed B-cell NHL patients. Aggressive NHL includes patients with histologic diagnoses of primarily diffuse large B cell (DLBL, DLBCL, or DLCL) (40% of all newly diagnosed patients have diffuse large cell), Burkitt's, and mantle cell. The clinical course of NHL is highly variable. A major determinant of clinical course is the histologic subtype. Most indolent types of NHL are considered to be incurable disease. Patients respond initially to either chemotherapy or antibody therapy and most will relapse. Studies to date have not demonstrated an improvement in survival with early intervention. In asymptomatic patients, it is acceptable to "watch and wait" until the patient becomes symptomatic or the disease pace appears to be accelerating. Over time, the disease may transform to a more aggressive histology. The median survival is 8 to 10 years, and indolent patients often receive 3 or more treatments during the treatment phase of their disease. Initial treatment of the symptomatic indolent NHL patient historically has been combination chemotherapy. The most commonly used agents include: cyclophosphamide, vincristine and prednisone (CVP); or cyclophosphamide, adriamycin, vincristine, prednisone (CHOP). Approximately 70% to 80% of patients will respond to their initial chemotherapy, duration of remissions last on the order of 2-3 years. Ultimately the majority of patients relapse. The discovery and clinical use of the anti-CD20 antibody, rituximab, has provided significant improvements in response and survival rate. The current standard of care for most patients is rituximab + CHOP (R-CHOP) or rituximab + CVP (R-CVP). Interferon is approved for initial treatment of NHL in combination with alkylating agents, but has limited use in the U.S. Rituximab therapy has been shown to be efficacious in several types of NHL, and is currently approved as a first line treatment for both indolent (follicular lymphoma) and aggressive NHL (diffuse large B cell lymphoma). However, there are significant limitations of anti-CD20 monoclonal antibody (mAb), including primary resistance (50% response in relapsed indolent patients), acquired resistance (50% response rate upon re-treatment), rare complete response (2% complete resonse rate in relapsed population), and a continued pattern of relapse. Finally, many B cells do not express CD20, and thus many B-cell disorders are not treatable using anti-CD20 antibody therapy.

In addition to NHL there are several types of leukemias that result from disregulation of B cells. Chronic lymphocytic leukemia (also known as "chronic lymphoid leukemia" or "CLL"), is a type of adult leukemia caused by an abnormal accumulation of B lymphocytes. In CLL, the malignant lymphocytes may look normal and mature, but they are not able to cope effectively with infection. CLL is the most common form of leukemia in adults. Men are twice as likely to develop CLL as women. However, the key risk factor is age. Over 75% of new cases are diagnosed in patients over age 50. More than 10,000 cases are diagnosed every year and the mortality is almost 5,000 a year (American

Cancer Society, 2006; and SEER Cancer Statistics Review). CLL is an incurable disease but progresses slowly in most cases. Many people with CLL lead normal and active lives for many years. Because of its slow onset, early-stage CLL is generally not treated since it is believed that early CLL intervention does not improve survival time or quality of life. Instead, the condition is monitored over time. Initial CLL treatments vary depending on the exact diagnosis and the progression of the disease. There are dozens of agents used for CLL therapy. Combination chemotherapy regimens such as FCR (fludarabine, cyclophosphamide and rituximab), and BR (bendamustine and rituximab) are effective in both newly-diagnosed and relapsed CLL. Allogeneic bone marrow (stem cell) transplantation is rarely used as a first-line treatment for CLL due to its risk.

Another type of leukemia is acute lymphoblastic leukemia (ALL), also known as acute lymphocytic leukemia. ALL is characterised by the overproduction and continuous multiplication of malignant and immature white blood cells (also known as lymphoblasts) in the bone marrow. 'Acute' refers to the undifferentiated, immature state of the circulating lymphocytes ("blasts"), and that the disease progresses rapidly with life expectancy of weeks to months if left untreated. ALL is most common in childhood with a peak incidence of 4-5 years of age. Children of age 12- 16 die more easily from it than others. Currently, at least 80% of childhood ALL are considered curable. Under 4,000 cases are diagnosed every year and the mortality is almost 1,500 a year (American Cancer Society, 2006; and SEER Cancer Statistics Review).

The human CD 19 molecule is a structurally distinct cell surface receptor expressed on the surface of human B cells, including, but not limited to, pre-B cells, B cells in early development {i.e., immature B cells), mature B cells through terminal differentiation into plasma cells, and malignant B cells. CD 19 is expressed by most pre-B acute lymphoblastic leukemias (ALL), non-Hodgkin's lymphomas, B cell chronic lymphocytic leukemias (CLL), pro-lymphocytic leukemias, hairy cell leukemias, common acute lymphocytic leukemias, and some Null-acute lymphoblastic leukemias (Nadler et al, J. Immunol., 131 :244-250 (1983), Loken et al, Blood, 70:1316-1324 (1987), Uckun et al, Blood, 71 :13-29 (1988), Anderson et al, 1984. Blood, 63:1424-1433 (1984), Scheuermann, Leuk. Lymphoma, 18:385-397(1995)). The expression of CD 19 on plasma cells further suggests it may be expressed on differentiated B cell tumors such as multiple myeloma, plasmacytomas, Waldenstrom's tumors (Grossbard et al., Br. J. Haematol, 102:509- 15(1998); Treon et al, Semin. Oncol, 30:248-52(2003)).

Therefore, the CD 19 antigen is a target for immunotherapy in the treatment of non-Hodgkin's lymphoma (including each the subtypes described herein), chronic lymphocytic leukemia and/or acute lymphoblastic leukemia.

Certain CD19 therapies have been shown. T cells expressing an anti-CD19 chimeric antigen receptor (CAR) including both CD3-ζ and the 4-BB costimulatory domain were administered to three patients with advanced CLL. Kalos et al., T cells with Chimeric Antigen Receptors Have Potent Antitumor Effects and Can Establish Memory in Patients with Advanced Leukemia, Science Translational Medicine, vol. 3, no. 95 (10 August 2011), which is incorporated by reference in its entirety. Sadelain et al., The promise and potential pitfalls of chimeric antigen receptors, Current Opinion in Immunology, Elsevier, vol. 21, no.2, 2 April 2009, which is incorporated by reference in its entirety, also describes anti-CD19 chimeric antigen receptors (CARs). Neither Kalos et al. nor Sadelain et al., however, describe the antibody specific for CD19 in combination with bendamustine as exemplified herein.

Bendamustine as a therapy in the treatment of non-hodgkin's lymphoma was described in Bremer et al., High rates of long lasting remission after 5-day bendamustine chemotherapy cycles in pre-treated low-grade non-Hodgkin's lymphomas, Journal of Cancer Research and Clinical Oncology, Springer International, Berlin, DE, vol. 128, no. 11, 1 November 2002, which is incorporated by reference in its entirety, and WO2006065392, which is incorporated by reference in its entirety, but neither suggests the antibody specific for CD19 in combination with bendamustine as exemplified herein.

The use of a CD19 antibody in non-specific B cell lymphomas is discussed in WO2007076950 (US2007154473), which are both incorporated by reference in their entireties, along with the cursory mention of bendamustine within a long list of potential combination partners, but fails either to teach the antibody exemplified herein or suggest the synergistic effects of the combination in the treatment of non-Hodgkin's lymphoma, chronic lymphocytic leukemia and/or acute lymphoblastic leukemia as exemplified herein.

The use of a CD19 antibody in CLL, NHL and ALL is described in Scheuermann et al., CD19 Antigen in Leukemia and Lymphoma Diagnosis and Immunotherapy, Leukemia and Lymphoma, Vol. 18, 385-397 (1995), which is incorporated by reference in its entirety, but fails to suggest the combination exemplified herein.

Additional antibodies specific for CD19 are described in WO2005012493 (US7109304), WO2010053716 (US12/266,999) (Immunomedics); WO2007002223 (US US8097703) (Medarex); WO2008022152 (12/377,251) and WO2008150494 (Xencor), WO2008031056 (US11/852,106) (Medimmune); WO 2007076950 (US 11/648,505 ) (Merck Patent GmbH); WO 2009/052431 (US12/253,895) (Seattle Genetics); and WO2010095031 (12/710,442) (Glenmark Pharmaceuticals), which are all incorporated by reference in their entireties.

Combinations of antibodies specific for CD19 and other agents are described in WO2010151341 (US 13/377,514) (The Feinstein Institute); US5686072 (University of Texas), and WO2002022212 (PCT/US01/29026) (IDEC Pharmaceuticals), which are all incorporated by reference in their entireties.

It is clear that in spite of the recent progress in the discovery and development of anti-cancer agents, many

forms of cancer involving CD19-expressing tumors still have a poor prognosis. Thus, there is a need for improved methods for treating such forms of cancer.

## Summary

Neither alone nor in combination does the prior art suggest the synergistic effects of the combination of the exemplified antibody and bendamustine in the treatment of non-Hodgkin's lymphoma, chronic lymphocytic leukemia and/or acute lymphoblastic leukemia.

In one aspect, the present disclosure relates to a synergistic combination of an antibody specific for CD19 and a nitrogen mustard. Such combinations are useful in the treatment of B cell malignancies, such as, non-Hodgkin's lymphoma, chronic lymphocytic leukemia and/or acute lymphoblastic leukemia.

In vitro and in vivo models are considered indicative of how a certain compound or combination of compounds would behave in humans. In addition, when compounds are combined either in vitro or in vivo, one expects that the combination has only additive effects. Surprisingly, the inventors found that the combination of a particular antibody specific for CD19 and bendamustine mediated a synergistic level of specific cell killing in a chronic B-cell leukemia cell line (MEC-1) in comparison to the antibody and bendamustine alone. This *in vitro* model is indicative of how the combination will work in the treatment of chronic lymphoid leukemia (CLL) in humans. In addition, and also unexpectedly, the inventors found that the combination of a particular antibody specific for CD19 and bendamustine inhibited tumor growth and synergistically increased median survival days and median increase in lifespan, both in Burkitt's lymphoma SCID mouse models, in comparison to the antibody and bendamustine alone. These *in vivo* models are indicative of how the combination will work in the treatment of non-Hodgkin's lymphoma in humans. In summary, the combination of the exemplified anti-CD19 antibody and bendamustine behaved synergistically in models relevant to NHL and CLL. As both NHL and CLL are B cell related disorders and CD19 is highly expressed on B-cells, the exemplified combination would have the same mechanism of action and should also behave synergistically in the treatment of other B cell related disorders, e.g. ALL.

Therefore, the combination of the exemplified antibody specific for CD19 and bendamustine will be effective in the treatment of humans in non-Hodgkin's lymphoma, chronic lymphocytic leukemia and/or acute lymphoblastic leukemia. In addition, the antibody specific to CD19 exemplified in the present specification has already entered into clinical trials, where such combinations can be confirmed in humans.

As the mechanism of action of bendamustine and other nitrogen mustards are similar, as they are alkylating agents that form interstrand cross-links (ICLs) between DNA bases, thus blocking fundamental processes such as replication and transcription, it is believed that synergy should also be seen when treating humans having non-Hodgkin's lymphoma, chronic lymphocytic leukemia and/or acute lymphoblastic leukemia with a combination of the exemplified anti-CD19 antibody and a nitrogen mustard other than bendamustine.

As the exemplified anti-CD19 antibody and other anti-CD19 antibodies bind CD19, it is believed that synergy should also be seen when treating humans having non-Hodgkin's lymphoma, chronic lymphocytic leukemia and/or acute lymphoblastic leukemia with a combination of any anti-CD19 antibody and a nitrogen mustard, e.g., bendamustine.

As the exemplified anti-CD19 antibody binds a specific epitope of CD19, it is believed that antibodies that cross-compete with the exemplified antibody or bind to the same epitope as the exemplified antibody should also behave synergistically when treating humans having non-Hodgkin's lymphoma, chronic lymphocytic leukemia and/or acute lymphoblastic leukemia when used in combination with a nitrogen mustard, e.g., bendamustine.

An aspect of the present disclosure comprises a synergistic combination wherein the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and bendamustine. In preferred aspects, the combination is used for the treatment of non-Hodgkin's lymphoma, chronic lymphocytic leukemia and/or acute lymphoblastic leukemia.

## Description of Drawings

**Figure** 1 shows the cytotoxicity effects of MOR00208 and bendamustine alone and in combination on MEC-1 cells.

**Figure** 2 shows the ADCC dose reponse curves of the combination of MOR00208 and bendamustine in MEC-1 cells.

**Figure** 3 shows the amino acid sequence of the variable domains of MOR00208.

**Figure** 4 shows the amino acid sequence of the Fc regions of MOR00208.

**Figure** 5 shows the normalized specific killing data of Table 2.

**Figure** 6 shows the results of the human Ramos Burkitt's B-cell lymphoma survival model in SCID mice as described in Example 3. The figure represents the data shown in Table 6, but excludes treatment related deaths.

**Figure** 7 shows the statistical analysis of the results of the subcutaneously (SC)-implanted human Ramos Burkitt's B-cell lymphoma tumor growth model in SCID mice, as described in Example 2.

**Figure** 8 shows the results of the subcutaneously (SC)-implanted human Ramos Burkitt's B-cell lymphoma tumor growth model in SCID mice, as described in Example 2.

**Figure** 9 shows the results of the subcutaneously (SC)-implanted human Ramos Burkitt's B-cell lymphoma tumor growth model in SCID mice, as described in Example 2. In this figure the BEN dosage is 13mg/kg.

**Figure 10** shows the results of the subcutaneously (SC)-implanted human Ramos Burkitt's B-cell lymphoma tumor growth model in SCID mice, as described in Example 2. In this figure the BEN dosage is 16mg/kg.

## Detailed description of the invention

"Synergy", "synergism" or "synergistic" mean more than the expected additive effect of a combination. The "synergy", "synergism" or "synergistic" effect of a combination is determined herein by the methods of Chou et al., Clarke et al. and/or Webb et al. See Ting-Chao Chou, Theoretical Basis, Experimental Design, and Computerized Simulation of Synergism and Antagonism in Drug Combination Studies, Pharmacol Rev 58:621-681 (2006), which is incorporated by reference in its entirety. See also Clarke et al., Issues in experimental design and endpoint analysis in the study of experimental cytotoxic agents in vivo in breast cancer and other models, Breast Cancer Research and Treatment 46:255-278 (1997), which is incorporated by reference in its entirety. See also Webb, J. L. (1963) Enzyme and Metabolic Inhibitors, Academic Press, New York, which is incorporated by reference in its entirety.

The term "antibody" means monoclonal antibodies, including any isotype, such as, IgG, IgM, IgA, IgD and IgE. An IgG antibody is comprised of two identical heavy chains and two identical light chains that are joined by disulfide bonds. Each heavy and light chain contains a constant region and a variable region. Each variable region contains three segments called "complementarity-determining regions" ("CDRs") or "hypervariable regions", which are primarily responsible for binding an epitope of an antigen. They are referred to as CDR1, CDR2, and CDR3, numbered sequentially from the N-terminus. The more highly conserved portions of the variable regions outside of the CDRs are called the "framework regions". An "antibody fragment" means an Fv, scFv, dsFv, Fab, Fab' F(ab')2 fragment, or other fragment, which contains at least one variable heavy or variable light chain, each containing CDRs and framework regions.

A "nitrogen mustard" is a nonspecific DNA alkylating agents used as chemotherapy. Alkylating agents add an alkyl group (CnH2n+1) to nucleic acid bases, e.g., adding an alkyl group to the guanine base of DNA at the number 7 nitrogen atom of the imidazole ring. The alkylation steps result in the formation of interstrand cross-links (ICLs). These ICLs are highly cytotoxic, since they block fundamental metabolic processes such as replication and transcription. Nitrogen mustards include cyclophosphamide, chlorambucil, uramustine, ifosfamide, melphalan and bendamustine.

Cyclophosphamide is marketed as Endoxan, Cytoxan, Neosar, Procytox, and Revimmune, and is also known as cytophosphane. Cyclophosphamide, or combinations including cyclophosphamide, is used in the treatment of lymphomas, leukemia and some solid tumors. Cyclophosphamide has the following structure:

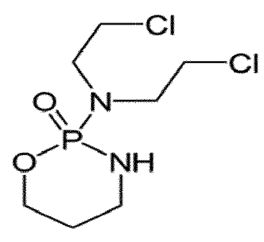

Chlorambucil is marketed as Leukeran by GlaxoSmithKline. It is used mainly in the treatment of chronic lymphocytic leukemia. Chlorambucil has the following structure:

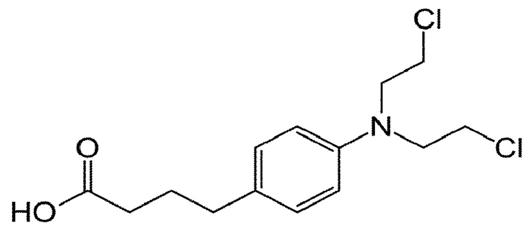

Uramustine is used in the treatment of non-Hodgkin's lymphoma. Uramustine has the following structure:

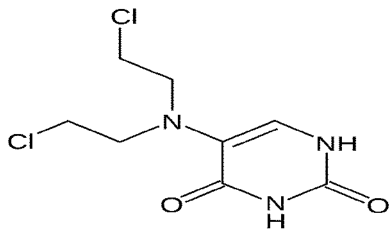

Ifosfamide is marketed as Mitoxana and Ifex. Ifosfamide has the following structure:

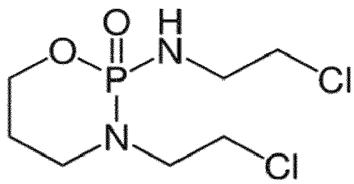

Melphalan is marketed as Alkeran. Melphalan has the following structure:

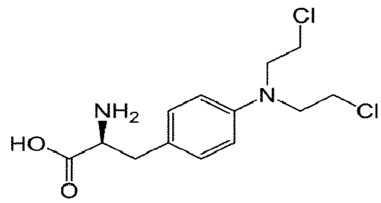

Bendamustine is marketed under the names Ribomustin®,and Treanda®, and is also known as SDX-105, by Mundipharma International Corporation Limited (Licensee of Astellas Pharma GmbH) and Cephalon for the treatment of chronic lymphocytic leukemias (CLL), indolent B-cell non-Hodgkin's lymphoma (NHL), and other lymphomas. Bendamustine has the following structure:

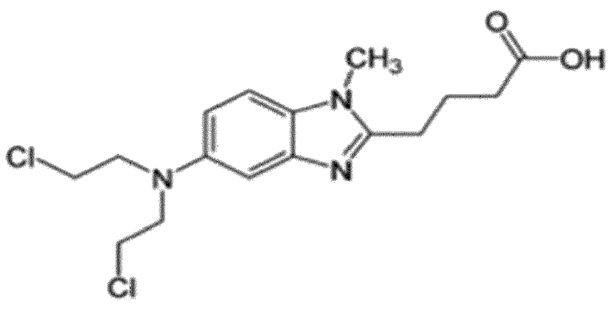

"BEN" when used herein means bendamustine.
"VH" refers to the variable region of an immunoglobulin heavy chain of an antibody, or antibody fragment. "VL" refers to the variable region of the immunoglobulin light chain of an antibody, or antibody fragment.

The term "CD19" refers to the protein known as CD19, having the following synonyms: B4, B-lymphocyte antigen CD19, B-lymphocyte surface antigen B4, CVID3, Differentiation antigen CD19, MGC12802, and T-cell surface antigen Leu-12.

Human CD19 has the amino acid sequence of:

MPPPRLLFFLLFLTPMEVRPEEPLVVKVEEGDNAVLQCLKGTSDGPTQQLTWSRESPLKPFLKLSL
GLPGLGIHMRPLAIWLFIFNVSQQMGGFYLCQPGPPSEKAWQPGWTVNVEGSGELFRWNVSDLG
GLGCGLKNRSSEGPSSPSGKLMSPKLYVWAKDRPEIWEGEPPCLPPRDSLNQSLSQDLTMAPGS
TLWLSCGVPPDSVSRGPLSWTHVHPKGPKSLLSLELKDDRPARDMWVMETGLLLPRATAQDAGK
YYCHRGNLTMSFHLEITARPVLWHWLLRTGGWKVSAVTLAYLIFCLCSLVGILHLQRALVLRRKRK
RMTDPTRRFFKVTPPPGSGPQNQYGNVLSLPTPTSGLGRAQRWAAGLGGTAPSYGNPSSDVQA
DGALGSRSPPGVGPEEEEGEGYEEPDSEEDSEFYENDSNLGQDQLSQDGSGYENPEDEPLGPE
DEDSFSNAESYENEDEELTQPVARTMDFLSPHGSAWDPSREATSLGSQSYEDMRGILYAAPQLR
SIRGQPGPNHEEDADSYENMDNPDGPDPAWGGGGRMGTWSTR. (SEQ ID NO: 7)

"MOR00208" is an anti-CD19 antibody. The amino acid sequence of the variable domains is provided in Figure 3. The amino acid sequence of the heavy and light chain Fc regions of MOR00208 are provided in Figure 4. "MOR00208" and "XmAb 5574" are used as synonyms to describe the antibody shown in Figures 3 and 4. The MOR00208 antibody is described in US patent application serial number 12/377,251, which is incorporated by reference in its entirety.

Additional antibodies specific for CD19 are described in US patent no. 7,109,304 (Immunomedics), which is incorporated by reference in its entirety; US application serial no. 11/917,750 (Medarex), which is incorporated by reference in its entirety; US application serial no. 11/852,106 (Medimmune), which is incorporated by reference in its entirety; US application serial no. 11/648,505 (Merck Patent GmbH), which is incorporated by reference in its entirety; US patent no. 7,968,687 (Seattle Genetics), which is incorporated by reference in its entirety; and US application serial no. 12/710,442 (Glenmark Pharmaceuticals), which is incorporated by reference in its entirety.

"Fc region" means the constant region of an antibody, which in humans may be of the IgG1, 2, 3, 4 subclass or others. The sequences of human Fc regions are available at IMGT, Human IGH C-REGIONs, http://www.imgt.org/IMGTrepertoire/Proteins /protein/human/IGH/IGHC/Hu_IGHCallgenes.html (retrieved on 16 May 2011).

"RefmAb33" is an antibody whose amino acid sequence is as follows:

Heavy chain including the Fc region:

QVTLRESGPALVKPTQTLTLTCTFSGFSLSTAGMSVGWIRQPPGKALEWLADIWWDDKKH
YNPSLKDRLTISKDTSKNQVVLKVTNMDPADTATYYCARDMIFNFYFDVWGQGTTVTVSSASTKG
PSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPELLGGPDVFLFPPKPKDTLMIS
RTPEVTCVVVDVSHEDPEVQFNWYVDGVEVHNAKTKPREEQFNSTFRVVSVLTVVHQDWLNGKE
YKCKVSNKALPAPEEKTISKTKGQPREPQVYTLPPSREEMTKNQVSLTCLVKGFYPSDIAVEWESN
GQPENNYKTTPPMLDSDGSFFLYSKLTVDKSRWQQGNVFSCSVMHEALHNHYTQKSLSLSPGK
(SEQ ID NO: 8)

Light chain including the Fc region:

DIQMTQSPSTLSASVGDRVTITCSASSRVGYMHWYQQKPGKAPKLLIYDTSKLASGVPSRF
SGSGSGTEFTLTISSLQPDDFATYYCFQGSGYPFTFGGGTKVEIKRTVAAPSVFIFPPSDEQLKSGT
ASVVCLLNNFYPREAKVQWKVDNALQSGNSQESVTEQDSKDSTYSLSSTLTLSKADYEKHKVYAC
EVTHQGLSSPVTKSFNRGEC (SEQ ID NO: 9)

RefmAb33 is specific for RSV, and is used as isotype control, as it shares the same Fc region as MOR00208.

A "combination" means more than one item, e.g. a compound such as an antibody and bendamustine.

The present disclosure also relates to combinations, pharmaceuticals, and pharmaceutical compositions containing the described combinations. The two components of the synergistic combination of the present invention, e.g. the antibody specific for CD19 and bendamustine, may be administered together, simultaneously or separately. When administered together, the two components may be formulated together in one pharmaceutical composition, which may include a pharmaceutical acceptable carrier or excipient. Alternatively the two components might also be formulated in

different pharmaceutical compositions. In this case the two components can be administered simultaneously or subsequently. In an embodiment, bendamustine, is administered prior to and/or separately from the administration of the antibody specific for CD19, e.g. MOR00208.

A pharmaceutical composition includes an active agent, eg. an antibody for therapeutic use in humans. A pharmaceutical composition may include acceptable carriers or excipients.

"Administered" or "administration" includes but is not limited to delivery by an injectable form, such as, for example, an intravenous, intramuscular, intradermal or subcutaneous route or mucosal route, for example, as a nasal spray or aerosol for inhalation or as an ingestable solution, capsule or tablet.

A "therapeutically effective amount" of a compound or combination refers to an amount sufficient to cure, alleviate or partially arrest the clinical manifestations of a given disease or disorder and its complications. The amount that is effective for a particular therapeutic purpose will depend on the severity of the disease or injury as well as on the weight and general state of the subject. It will be understood that determination of an appropriate dosage may be achieved, using routine experimentation, by constructing a matrix of values and testing different points in the matrix, all of which is within the ordinary skills of a trained physician or clinical scientist.

The "CDRs" herein are defined by either Chothia et al or Kabat et al. See Chothia C, Lesk AM. (1987) Canonical structures for the hypervariable regions of immunoglobulins. J Mol Biol., 196(4):901-17, which is incorporated by reference in its entirety. See Kabat E.A, Wu T.T., Perry H.M., Gottesman K.S. and Foeller C. (1991). Sequences of Proteins of Immunological Interest. 5th edit., NIH Publication no. 91-3242, US Dept. of Health and Human Services, Washington, DC, which is incorporated by reference in its entirety.

"Cross competes" means the ability of an antibody or other binding agent to interfere with the binding of other antibodies or binding agents to CD19 in a standard competitive binding assay. The ability or extent to which an antibody or other binding agent is able to interfere with the binding of another antibody or binding molecule to CD19, and, therefore whether it can be said to cross-compete according to the invention, can be determined using standard competition binding assays. One suitable assay involves the use of the Biacore technology (e.g. by using the BIAcore 3000 instrument (Biacore, Uppsala, Sweden)), which can measure the extent of interactions using surface plasmon resonance technology. Another assay for measuring cross-competing uses an ELISA-based approach. A high throughput process for "epitope binning" antibodies based upon their cross-competition is described in International Patent Application No. WO 2003/48731

The term "epitope" includes any protein determinant capable of specific binding to an antibody or otherwise interacting with a molecule. Epitopic determinants generally consist of chemically active surface groupings of molecules such as amino acids or carbohydrate or sugar side chains and can have specific three-dimensional structural characteristics, as well as specific charge characteristics. An epitope may be "linear" or "conformational." The term "linear epitope" refers to an epitope with all of the points of interaction between the protein and the interacting molecule (such as an antibody) occur linearally along the primary amino acid sequence of the protein (continuous). The term "conformational epitope" refers to an epitope in which discontinuous amino acids that come together in three dimensional conformation. In a conformational epitope, the points of interaction occur across amino acid residues on the protein that are separated from one another.

"Binds the same epitope as" means the ability of an antibody or other binding agent to bind to CD19 and having the same epitope as the exemplified antibody. The epitopes of the exemplified antibody and other antibodies to CD19 can be determined using standard epitope mapping techniques. Epitope mapping techniques, well known in the art. include Epitope Mapping Protocols in Methods in Molecular Biology, Vol. 66 (Glenn E.Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871 ; Geysen et al, (1984) Proc. Natl. Acad. Sci. USA 8:3998-4002; Geysen et al, (1985) Proc. Natl. Acad. Sci. USA 82:78-182; Geysen et al, (1986) Mol. Immunol. 23 :709-715. Similarly, conformational epitopes are readily identified by determining spatial conformation of amino acids such as by, e.g., hydrogen/deuterium exchange, x-ray crystallography and two-dimensional nuclear magnetic resonance. See, e.g., Epitope Mapping Protocols, supra. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al, (1981) Proc. Natl. Acad. Sci USA 78:3824-3828; for determining antigenicity profiles, and the Kyte-Doolittle technique, Kyte et al, (1982) J.Mol. Biol. 157: 105-132; for hydropathy plots.

## Embodiments

An aspect of the present disclosure comprises a combination of an antibody specific for CD19 and a nitrogen mustard for use in the treatment of non-Hodgkin's lymphoma, chronic lymphocytic leukemia and/or acute lymphoblastic leukemia. In embodiments, the combination is synergistic.

Herein, the combination of the exemplified anti-CD19 antibody and bendamustine behaved synergistically in *in vitro* and *in vivo* models relevant to NHL and CLL. As both NHL and CLL are B cell related disorders and CD19 is highly expressed on B-cells, the exemplified combination should have the same mechanism of action and should also behave synergistically in the treatment of other B cell related disorders, e.g. ALL. Therefore, the combination of the exemplified antibody specific for CD19 and bendamustine will be effective in the treatment of humans in non-Hodgkin's lymphoma, chronic lymphocytic leukemia and/or acute lymphoblastic leukemia.

As the mechanism of action of bendamustine and other nitrogen mustards are similar, as they are alkylating agents that form interstrand cross-links (ICLs) between DNA bases, thus blocking fundamental processes such as replication and transcription, it is believed that synergy should also be seen when treating humans having non-Hodgkin's lymphoma, chronic lymphocytic leukemia and/or acute lymphoblastic leukemia with a combination of the exemplified anti-CD19 antibody and a nitrogen mustard other than bendamustine, e.g. cyclophosphamide, chlorambucil, uramustine, ifosfamide, and melphalan.

As the exemplified anti-CD19 antibody and other anti-CD19 antibodies bind CD19, it is believed that synergy should also be seen when treating humans having non-Hodgkin's lymphoma, chronic lymphocytic leukemia and/or acute lymphoblastic leukemia with a combination of any anti-CD19 antibody and a nitrogen mustard, where the anti-CD19 antibody is, for example, described in US patent application serial number 12/377,251 (Xencor), WO2005012493, WO2010053716 (Immunomedics); WO2007002223 (Medarex); WO2008022152 (Xencor); WO2008031056 (Medimmune); WO 2007/076950 (Merck Patent GmbH); WO 2009/052431 (Seattle Genetics); and WO2010095031 (Glenmark Pharmaceuticals), all of which are incorporated by reference in their entireties.

In embodiments, the antibody specific for CD19 comprises an antibody that cross-competes with the antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

In embodiments, the antibody specific for CD19 comprises an antibody that binds to the same epitope as an antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

In embodiments, the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

In embodiments, the antibody specific for CD19 comprises a variable heavy chain of the sequence EVQLVESGGGLVKPGGSLKLSCAASGYTFTSYVMHWVRQAPGKGLEWIGYINPY NDGTKYNEKFQGRVTISSDKSISTAYMELSSLRSEDTAMYYCARGTYYYGTRVFDYWG QGTLVTVSS (SEQ ID NO: 10) and a variable light chain of the sequence DIVMTQSPATLSLSPGERATLSCRSSKSLQNVNGNTYLYWFQQKPGQSPQLLIYR MSNLNSGVPDRFSGSGSGTEFTLTISSLEPEDFAVYYCMQHLEYPITFGAGTKLEIK (SEQ ID NO: 11).

In embodiments, the antibody specific for CD19 comprises a heavy chain constant domain of the sequence

ASTKGPSVFPLAPSSKSTSGGTAALGCLVKD YFPEPVTVSWNSGALTSGVH
TFPAVLQSSGLYSLSSWTVPSSSLGTQTYICNVNHKPSNTKVDKKVEPKSCDKTHTCPPCPAPELL
GGPDVFLFPPKPKDTLMISRTPEVTCVVVDVSHEDPEVQFNWYVDGVEVHNAKTKPREEQFNSTF
RVVSVLTVVHQDWLNGKEYKCKVSNKALPAPEEKTISKTKGQPREPQVYTLPPSREEMTKNQVSL
TCLVKGFYPSDIAVEWESNGQPENNYKTTPPMLDSDGSFFLYSKLTVDKSRWQQGNVFSCSVMH
EALHNHYTQKSLSLSPGK. (SEQ ID NO: 12)

In embodiments, the antibody specific for CD19 comprises a light chain constant domain of the sequence

RTVAAPSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC. (SEQ ID NO: 13)

In embodiments, the nitrogen mustard is bendamustine.

In embodiments, the components of the combination, the antibody specific for CD19 and bendamustine, are administered separately. In an embodiment, bendamustine is administered prior to administration of the antibody specific for CD19.

In embodiments the combination is a pharmaceutical composition. In embodiments, the composition comprises an acceptable carrier. In embodiments, the combination is administered in an effective amount.

In another aspect the synergistic combination of an antibody specific for CD19 comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and bendamustine is able to mediate killing of MEC-1 cells by ADCC in the presence of isolated human PBMCs with an at least two-fold, three-fold, four-fold, or five-fold better efficacy than bendamustine alone.

An aspect of the present disclosure comprises a synergistic combination of an antibody specific for CD19 comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and bendamustine for the treatment of non-Hodgkin's lymphoma, chronic lymphocytic leukemia and/or acute lymphoblastic leukemia. In embodiments, the non-Hodgkin's lymphoma is selected from the group consisting of follicular lymphoma, small lymphocytic lymphoma, mucosa-associated lymphoid tissue, marginal zone, diffuse large B cell, Burkitt's, and mantle cell.

Another aspect comprises a method of treating non-Hodgkin's lymphoma, chronic lymphocytic leukemia and/or acute lymphoblastic leukemia in an individual in need thereof, which method comprises administration of an antibody specific for CD19 and a nitrogen mustard. In embodiments of the method, the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6). In embodiments of the method, the antibody comprises the exemplified antibody specific for CD19. In embodiments of the method the nitrogen mustard is bendamustine.

## Examples

Example 1: Inhibition of proliferation of MEC-1 cells using MOR00208 and bendamustine alone and in combination

Materials

MEC-1 cells: chronic B-cell leukemia cell line DSMZ# ACC497; Cell Medium: Iscove's Modified Dulbecco's Medium (IMDM) with GlutaMAX™, Invitrogen, Cat No.: 31980-048, 20% FCS; PBMCs: RPMI1640, with stabile Glutamine, PAN Biotech GmbH, Cat No.: P04-13500 supplemented with 10% FCS; Biocoll: Biochrome AG CAT No.: L6115 LOT No.: 1050T; Bendamustine: Mundipharma LOT No.: 88018; FCS: PAN CAT No.: 3302-P282403 LOT No.: P282403; and RefmAb33 (anti-RSV) with same Fc region as MOR00208.

Methods

The cytotoxicity of MOR00208 and bendamustine alone and in combination was tested in MEC-1 cells. BEN is an alkylating agent, therefore, functions via direct cytoxicity in MEC-1 cells. MOR00208 targets CD19 and additionally functions via ADCC in killing MEC-1 cells. For the following groups MEC-1 cell killing was measured: BEN at 100μg/ml; MOR00208 at 6,6pm and the combination of MOR00208 at 6,6pm and BEN at 100μg/ml. These concentrations were chosen as they are near or at the EC50 for MOR00208 and BEN. The following were used as controls: RefmAb33, or PBMCs alone. In both the BEN group and MOR00208+BEN combination group, MEC-1 cells were pre-incubated with BEN 48 hours prior to the ADCC assay measurements. The MEC-1 cells were stained using 1mg/ml Calcein AM then counted and adjusted to $2\times10^5$/ml. The PBMCs were counted and adjusted to $6\times10^6$/ml. The cell killing assays were done as follows: using 96 well plates, a 100μl cell suspension of MEC-1 cells was added per well, then 100μl cell suspension of PBMCs was added to each well resulting in an E:T ratio of 30:1. The antibodies were diluted to 1μg/ml in medium. Cells were centrifuged and re-suspended. To the target:effector cell-pellet, 100μl antibody solution or according control solution was added. The mixture was incubated for 4h in CO2-incubator at 37°C. The cell killing measurements were taken as follows: the incubated cell solution (~100μl) was transfered into FACS tubes and 200μl FACS buffer (DPBS + 3%FCS) and 0,5 μl PI stock solution was added to each tube. FACS-Calibur was used. Dead MEC-1 cells were stained with propidium iodide. Table 1 and Figure 1 show the raw data.

Table 1

| | Control | MOR00208 6,6pm | BEN 100 μg/ml | BEN+MOR00208 combination |
|---|---|---|---|---|
| Experiment 1 | 25,2 | 73,6 | 83,6 | 94,0 |
| Experiment 2 | 18 | 41,5 | 53,3 | 64,9 |
| Experiment 3 | 30,9 | 57,2 | 75,6 | 83,6 |

The values represent % dead cells. Each experiment represents PBMCs from different donors. The controls used for each experiment was RefMab33.

Table 2 shows the raw data of Table 1 normalized for specific killing and the results of the Chou calculations done in the determination of synergism.

Table 2

| | **Bendamustine μg/ml** | **MOR00208 6.6 pM** | **Ben+MOR00208 (combination)** | **Chou Index** |
|---|---|---|---|---|
| Experiment 1 | 0.85 | 0.70 | 1.0 | 0.2 |
| Experiment 2 | 0.75 | 0.50 | 1.0 | 0.7 |
| Experiment 3 | 0.85 | 0.50 | 1.0 | 0.8 |
| **Average** | 0.8 | 0.6 | 1.0 | 0.6 |

The values shown in Table 2 are calculated as follows: 1) from the raw data (% dead cells) shown in Table 1, the background (controls) were subtracted, resulting in the specific killing for each treatment group; then 2) the specific killing values were normalized by setting the combination of MOR00208 + BEN to 1. The averages of Table 2 are depicted in Figure 5. Example ADCC dose response curves used in the Chou factor calculations of the MOR00208 + BEN combination are shown in Figure 2.

Chou Index (CI) calculations were completed in order to determine synergy of the combination of the exemplified anti-CD19 antibody and bendamustine as compared to MOR00208 and BEN alone. Such calculations are described in Ting-Chao Chou, Theoretical Basis, Experimental Design, and Computerized Simulation of Synergism and Antagonism in Drug Combination Studies, Pharmacol Rev 58:621-681 (2006), which is incorporated by reference in its entirety and Chou TC, Talalay P, Quantitative analysis of dose-effect relationships: the combined effects of multiple drugs or enzyme inhibitors. Adv Enzyme Regul 22: 27-55 (1984), which is incorporated by reference in its entirety. The methods of Chou-Talalay are carried out using the CI-isobol method.

Median-effect equation

The median-effect equation models of the effect of an inhibitor (such as a drug) as $F_a/F_u$ =(D/D50)^m, where D is the dose, $F_a$ and $F_u$ is the fraction of the system affected and unaffected by the dose D ($F_a + F_u = 1$); D50 is the dose producing the median effect (e.g. IC50, ED50, LD50). The constant m determines the shape of the dose-effect curve. We used Excel Fit software to carry out a linear regression calculation to estimate the parameters m and D50.

The effects of the combination on MEC-1 cells is measured % cell death as described above. We define the fraction $F_u$ to be the ratio of % cell death of the treated cell line to the % cell death of the cell line exposed to a control. That is:

$$F_u = \%\ \text{cell death(treated cell line)} / \%\ \text{cell death (non-treated cell line)}$$

Then the % cell death of a cell line is the constant D50 in the median effect equation, which can be estimated by the linear regression described above.

CI-isobol method

The CI-isobol method provides a quantitative assessment of synergism between drugs. A combination index (CI) is estimated from dose-effect data of single and combined drug treatments. A value of CI less than 1 indicates synergism; CI = 1 indicates additive effect; and CI > 1 indicates antagonism. Drug interaction (synergism or antagonism) is more pronounced the farther a CI value is from 1.

Formally, the combination index (CI) of a combined drug treatment is defined as

$$CI = D_1/D_{x1} + D_2/D_{X2}$$

Here D1 and D2 are the doses of drug 1 and drug 2 of the combination, respectively; and Dx1, and Dx2 is the dose of a treatment with only drug 1 and drug 2 that would give the same effect as that of the combination. The doses Dx1 and Dx2 need to be estimated from the dose-effect data of single drug treatments. Essentially, a median effect equation is fitted to the data of each drug. From the median effect equation of a drug, we can estimate the dose (i.e. D) necessary to produce an effect (i.e. Fa, Fu). The further a point lies from the additive line, the bigger the different between 1 and its CI, thus the stronger the (synergistic or antagonistic) effect is.

Results

As shown in Table 2, the Chou index values indicate clear synergism of the combination of MOR00208 and bendamustine in the specific killing of MEC-1 cells as compared to MOR00208 and bendamustine alone. This conclusion is based upon the Chou calculations of 0,2, 0.7 and 0.75 of each of the three experiments, respectively, having an average of 0,6, where a CI <1 indicates synergism. Therefore, the combination of MOR00208 and bendamustine will also behave synergistically in the treatment of non-Hodgkin's lymphoma (NHL), chronic lymphoid leukemia (CLL), and acute lymphoblastic leukemia (ALL) in humans. In order to confirm the results of the above Chou calculations, the normalized data of Table 2 was evaluated for statistical significance using the Bonferroni's Multiple Comparison Test. See James, et al, Antibody-mediated B-cell depletion before adoptive immunotherapy with T cells expressing CD20-specific chimeric T-cell receptors facilitates eradication of leukemia in immunocompetent mice, Blood, 114(27):5454-63 (Epub 2009 Oct 30), which is incorporated by reference in its entirety. The results are shown in Table 3.

Table 3

| Bonferroni's Multiple Comparison Test | Mean Diff. | T value | Significant? (P < 0.05) | Summary |
|---|---|---|---|---|
| Bendamustine (100μg/ml) vs. BEN + MOR 208 combination | -0.1834 | 2.997 | Yes | * |
| MOR00208 (6.6pM) vs. BEN + MOR00208 combination | -0.4321 | 7.060 | Yes | *** |
| ** p < 0,05<br>*** p < 0,001 | | | | |

Results

As shown in Table 3, the Bonferroni's Multiple Comparison Test shows that the combination treatment of BEN + MOR00208 is statistically more effective in the specific killing of MEC-1 cells than the treatment of BEN and MOR00208 alone.

Example 2: MOR00208 and BEN alone and in combination in subcutaneously (SC)-implanted human Ramos Burkitt's B-cell lymphoma tumor growth model.

Materials

RAMOS human Burkitt's lymphoma cells (ATCC number CRL-1596, lot# 3953138); Vehicle control: 150 mM NaCl, 25 mg/mL mannitol, pH 5.5-6.0; (adjusted with 0.01 M NaOH). Ref_mAb_33_IgG_Xen (10 mg/mL in PBS, referred to as Ref_mAb_33). Six-week-old, female, C.B-17 SCID mice (CB17/Icr-Prkdcscid/IcrIcoCrl) were purchased from Charles River Laboratories (Wilmington, MA) and acclimated in the laboratories for nine days prior to experimentation.

Methods

SCID mice were implanted sub-cutaneously with RAMOS cells (~5 $\times$ $10^6$ cells/mouse). When the mice had tumors of approximately 150 mm3 in size, or -14 days after inoculation, they were separated into groups, where each group had tumor volumes of relatively the same size. Treatments began on Day 15. The treatment regimens are provided in Table 4. The study duration was 60 days.

Table 4

| Group No. | No. of Animals | Test Articles | Dose (mg/kg) | Treatment Route and Schedule |
|---|---|---|---|---|
| A/B | 10 | Bendamustine | 13, and 16 | IP, Q1Dx5 |
| D | 10 | MOR00208 | 6/10 | IV, 6 mg/kg Q3Dx2; 10mg/kg Q3Dx2/3 wks starting on Day 22 |
| E | 10 | Vehicle/ Ref_mAb_33 | 6/10 | IP, Q1Dx5 IV, 6 mg/kg Q3Dx2 ; 10 mg/kg Q3Dx2/3 wks starting on day 22 |
| F/G | 10 | MOR00208/ Bendamustine | 6 or 10/13 and 6 or 10/16 | MOR00208 and BEN as above |

Due to a technician error MOR00208 on Day 18 was not administered.

MOR00208, and bendamustine, were administered in a volume of 0.1 mL/10 g of body weight. MOR00208 and vehicle control/Ref_mAb_33 at a concentration of 0.6/1.0 mg/mL, and bendamustine at concentration of 1.3,and 1.6 mg/mL.

The readouts were 1) Median days to reach 4000 mg in size, where the statistical analysis was done using the log rank test and 2) Tumor size on study day 34, where the statistical analysis was done using the One-Way-ANOVA and Bonferroni's post hoc tests. (Raw data not shown). Tumor weights were calculated using the equation (l x w2)/2, where l and w refer to the larger and smaller dimensions collected at each measurement. The results are shown in Figures 7-10. The combination therapy was not significantly superior to the respective monotherapies in this subcut model, as compared to the clear synergy shown in the orthotopic survival model below. This is considered to be related to the ineffective MOR00208 dosing regimen in this model. The orthotopic survival model described below, however, is believed to be more predictive of how well the combination treatment would work in the treatment of CLL, NHL, and ALL in humans, as the orthotopic model better mimics the multifocal disease nature, including an involvement of the vascular system, as compared to the subcut, solid tumor model above.

Example 3 MOR00208 and bendamustine alone and in combination in human Non-hodgkin RAMOS tumor in SCID mice, survival model

Materials

Cyclophosphamide (Baxter, Lot. No.1A548C); Vehicle Control: 0.9% sodium chloride, 25mg/ml mannitol, pH 6.5-6.8 (adjusted with 0.01 M NaOH); SCID Mice (University of Adelaide, Waite Campus, Urrbaraie, SA, Australia, Strain C.B.-17-Igh-$1^{b}$-$Prkdc^{scid}$); RAMOS human Burkitt's lymphoma cells (ATCC number CRL-1596); Ref_mAb_33_IgG_Xen (10 mg/mL in PBS, referred to as Ref_mAb_33); Bendamustine (Mundipharma, Lot No. 83889).

Methods

SCID mice were pre-treated with Cyclophosphamide (75 mg/kg, i.p., twice daily) for two days prior to RAMOS cell inoculation (Day -2 and -1). On the day of inoculation (Day 0), the mice were separated into seven groups of ten mice each, and inoculated with $1 \times 10^6$ RAMOS cells each intravenously into the tail vein. The planned dosing regimen for each group is shown in Table 5 and commenced on Day 3. The study duration was 60 days.

Table 5: Dosing regimen

| Group | Compound | Treatment | Schedule |
|---|---|---|---|
| 2 and 3 | Bendamustine | 13/16 mg/kg, i.p, in 10 mL/kg | Once daily (Days 5-9) Twice weekly for 3 |
| 1 | MOR00208 | 3 mg/kg, i.v., in 10 mL/kg | weeks (Days 3, 6, 10, 13, 17 and 20) |
| 10 | Vehicle Control | i.p., 10 mL/kg | Once daily (Days 5-9) |
| 5 and 6 | Bendamustine /MOR00208 | 13/16 mg/kg, i.p; 3 mg/kg, i.v. in 10 mL/kg; | Once daily (Days 5-9) /twice weekly for 3 weeks (Days 3, 6, 10, 13, 17 and 20) |
| 4 | Bendamustine | 26 mg/kg, i.p, in 10 mL/kg | Once daily (Days 5-9) |
| 10 | Ref mAb | 3 mg/kg, i.v. | Day 3, 6, 10, 13, 17 and 20 |

The survival data is shown in Table 6 and Figure 6.

Table 6: Death of mice

| Group | Compound | Treatment | Death of Mice over the Course of Study [Day post Inoculation] |
|---|---|---|---|
| 1 | MOR00208 | 3 mg/kg, i.v. | 25; 29; 29; 30; 31; 33; 35; 38; 38; 39 |
| 2 | Bendamustine | 13 mg/kg, i.p. | 10*; 21; 21; 23; 24; 24; 24; 24; 25; 26 |
| 3 | Bendamustine | 16 mg/kg, i.p. | 24; 24; 24; 24; 24; 24; 25; 26; 26; 27 |
| 4 | Bendamustine | 26 mg/kg, i.p. | 10*; 10*; 10*; 10*; 10*; 12*; 12*; 14*; 16*; 23 |
| 5 | Bendamustine/ MOR00208 | 13/3 mg/kg, i.p. / i.v. | 12*; 30; 33; 33; 35; 40; 45; 45; 56; 56 |
| 6 | Bendamustine/ MOR00208 | 16/3 mg/kg, i.p. / i.v. | 33; 35; 38; 39; 40; 40; 45; 45; 45; 45 |
| 10 | Vehicle/ Ref_mAb | i.p. / 3 mg/kg, | 24; 24; 25; 25; 25; 26; 26; 26; 26; 29 |
| * Compound toxicity related death | | | |

From the raw data shown in Table 6, both the median survival in days and median increase in lifespan were calculated. All treatment related deaths were excluded in the calculations. The results are shown in Table 7.

Table 7

| Group | Treatment | **Median** Survival (Days Post-Inoculation) | Median % Increase in Lifespan **(ILS)**§ | Evaluation of combinatorial effects |
|---|---|---|---|---|
| 1 | MOR00208 | 32[a] | 25.5 | n.a. |
| 2 | Bendamustine 13 mg/kg | 24[b] | -5.88 | n.a. |
| 3 | Bendamustine 16 mg/kg | 24[c] | -5.88 | n.a. |
| 4 | Bendamustine 26 mg/kg | n.a. | n.a. | n.a. |
| 5 | Bendamustine/ MOR00208 13/3 mg/kg | 40[d] | 56.86 | Synergy/Potentiation* |
| 6 | Bendamustine/ MOR00208 16/3 mg/kg | 40[d] | 56.86 | Synergy/Potentiation** |
| 10 | Vehicle/ Ref_mAb 3 mg/kg | 25.5 | n.a. | n.a. |

a significantly different to Vehicle control/ Ref_mAb_33 (Group 10) (p<0.001), Bendamustine at 13 mg/kg (Group 2) (p<0.001), Bendamustine/ MOR00208 at 13/3 mg/kg (Group 5) (p<0.05) and Bendamustine/ MOR00208 at 16/3 mg/kg (Group 6) (p<0.001).
b significantly different to Vehicle control/ Ref_mAb_33 (Group 10) (p<0.05) and Bendamustine/ MOR00208 at 13/3 mg/kg (Group 5) (p<0.001).
c significantly different to Bendamustine/ MOR00208 at 16/3 mg/kg (Group 6) (p<0.001).
d significantly different to Vehicle Control/ Ref_mAb_33 (Group 10) (p<0.001).
§ vs. vehicle control/ Ref_mAb_33
* Synergy/Potentiation vs. the respective monotherapy groups as ILSCombo (56.86%) > ILSMOR00208 3mg/kg + ILSBendamustine 13mg/kg (25.5% + (-5.88)% = 19.62%)
** Synergy/Potentiation vs. the respective monotherapy groups as ILSCombo (56.86%) > ILSMOR00208 3mg/kg + ILSBendamustine 16mg/kg (25.5% + (-5.88)% = 19.62%).

Median % Increased Lifespan (ILS) is calculated as follows:

$$\text{Mean \% Increase in Lifespan} = (\text{Survival}_{\text{Treatment}} - \text{Mean Survival}_{\text{Control}}) / \text{Mean Survival}_{\text{Control}} * 100.$$

Survival times are measured in days post-inoculation.

Classification of Combinatorial Effects

The classification of the MOR000208/Bendamustine combination therapy (combo) effect was evaluated by comparing the ILS of the combination with the added ILS of the respective monotherapies:
Synergy/Potentiation*: ILSCombo > ILSMOR00208 3mg/kg + ILSBendamustine. Synergistic effects are classified as potentiation if at least one of the monotherapies has no effect. Additivity: ILSCombo = ILSMOR00208 3mg/kg + ILSBendamustine. Antagonism: ILSCombo < ILSMOR00208 3mg/kg + ILSBendamustine.

In addition to an analysis of the data for purposes of identifying synergy, the following statistical analysis was also completed. Statistical analyses were carried out using the median values. Any animal that died unexpectedly or was culled prior to Day 17 of the study in the Test Article treatment groups was excluded from survival analysis calculation. The death/ culling of these animals was attributed to compound toxicity rather than disease progression as they occurred well in advance of the first deaths in the Vehicle Control animals. A survival curve was created using the product limit of Kaplan and Meier, and survival curves compared using the log-rank (Mantel-Cox) test. Where significant differences were found, All Pairwise Multiple Comparison (Holm-Sidak Test) was performed. Comparison was done between all groups. In addition the comparison of the following groups were summarised in separate figures for each test article: Vehicle Control/ Ref_mAb (group 10) against Bendamustine groups (Groups 2, 3 and 4) and Vehicle Control / Ref_mAb (group 10) against Combination groups (Groups 5 and 6) or respective MOR00208 monotherapy group (group 1). A p value of less than 0.05 was considered significant. Results are shown in Tables 8-10.

Table 8:

**Vehicle Control, MOR00208 and Bendamustine Monotherapy:**
Log-rank (Mantel-Cox) Test: There is a significant difference ($p<0.001$).
All Pairwise Multiple Comparison Procedure (Holm-Sidak method):

| Group | Treatment | Group 1 | Group 2 | Group 3 |
|---|---|---|---|---|
| 10 | Vehicle Control/ Ref_mAb (3 mg/kg) | ***Yes | *Yes | No |
| 1 | MOR00208 (3 mg/kg) | | ***Yes | ***Yes |
| 2 | Bendamustine (13 mg/kg) | | | No |
| 3 | Bendamustine (16 mg/kg) | | | |

***Yes: There is a statistically significant difference ($p<0.001$).
*Yes: There is a statistically significant difference ($p<0.05$).
No: There is no statistically significant difference ($p\geq 0.05$).

Table 9:

**Vehicle Control, MOR00208/ Bendamustine Combination -Therapy and respective Monotherapy:**
Log-rank (Mantel-Cox) Test: There is a significant difference ($p<0.001$).
All Pairwise Multiple Comparison Procedure (Holm-Sidak method):

| Group | Treatment | Group 1 | Group 5 | Group 2 |
|---|---|---|---|---|
| 10 | Vehicle Control/ Ref_mAb (3 mg/kg) | ***Yes | ***Yes | *Yes |
| 1 | MOR00208 (3 mg/kg) | | *Yes | ***Yes |
| 5 | MOR00208/ Bendamustine (3/13 mg/kg) | | | ***Yes |
| 2 | Bendamustine (13 mg/kg) | | | |

***Yes: There is a statistically significant difference ($p<0.001$).
*Yes: There is a statistically significant difference ($p<0.05$).

Table 10:

| Group | Treatment | Group 1 | Group 6 | Group 3 |
|---|---|---|---|---|
| **Vehicle Control, MOR00208/ Bendamustine Combination -Therapy and respective Monotherapy:** Log-rank (Mantel-Cox) Test: There is a significant difference (p<0.001). All Pairwise Multiple Comparison Procedure (Holm-Sidak method): | | | | |
| 10 | Vehicle Control/ Ref_mAb (3 mg/kg) | ***Yes | ***Yes | No |
| 1 | MOR00208 (3 mg/kg) | | ***Yes | ***Yes |
| 6 | MOR00208/ Bendamustine (3/16 mg/kg) | | | ***Yes |
| 3 | Bendamustine (16 mg/kg) | | | |

***Yes: There is a statistically significant difference (p<0.001).
No: There is no statistically significant difference (p≥0.05).

Results

As shown in Tables 7-10 and Figure 6, the combination of MOR00208 and bendamustine behaved synergistically and was statistically significant in the Non-hodgkin RAMOS orthotopic tumor survival model as compared to MOR00208 and bendamustine alone.

It is to be understood that the description, specific examples and data, while indicating exemplary embodiments, are given by way of illustration and are not intended to limit the present invention. Various changes and modifications within the present invention will become apparent to the skilled artisan from the discussion, disclosure and data contained herein, and thus are considered part of the invention.

The invention further provides the following non-limiting embodiments:

1. A synergistic combination of an antibody specific for CD19 comprising an antibody that cross-competes with an antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and bendamustine for use in the treatment of non-Hodgkin's lymphoma, chronic lymphocytic leukemia and/or acute lymphoblastic leukemia.

2. A combination according to embodiment 1, wherein the antibody comprises an antibody that binds to the same epitope as an antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

3. A combination according to embodiment 1 or 2, wherein the antibody comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

4. A combination according to any one of the preceding embodiments, wherein the antibody comprises a variable heavy chain of the sequence EVQLVESGGGLVKPGGSLKLSCAASGYTFTSYVMHWVRQAPGKGLEWIGYINPY NDGTKYNEKFQGRVTISSDKSISTAYMELSSLRSEDTAMYYCARGTYYYGTRVFDYWG QGTLVTVSS (SEQ ID NO: 10) and a variable light chain of the sequence

DIVMTQSPATLSLSPGERATLSCRSSKSLQNVNGNTYLYWFQQKPGQSPQLLIYR MSNLNSGVPDRFSGSGSGTEFTLTISSLEPEDFAVYYCMQHLEYPITFGAGTKLEIK (SEQ ID NO: 11).

5. A combination according to any one of the preceding embodiments, wherein the antibody comprises a heavy chain constant domain of the sequence

ASTKGPSVFPLAPSSKSTSGGTAALGCLVKD YFPEPVTVSWNSGALTSGVH
TFPAVLQSSGLYSLSSWTVPSSSLGTQTYICNVNHKPSNTKVDKKVEPKSCDKTHTCPPCPAPELL
GGPDVFLFPPKPKDTLMISRTPEVTCVVVDVSHEDPEVQFNWYVDGVEVHNAKTKPREEQFNSTF
RVVSVLTVVHQDWLNGKEYKCKVSNKALPAPEEKTISKTKGQPREPQVYTLPPSREEMTKNQVSL
TCLVKGFYPSDIAVEWESNGQPENNYKTTPPMLDSDGSFFLYSKLTVDKSRWQQGNVFSCSVMH
EALHNHYTQKSLSLSPGK (SEQ ID NO: 12).

6. A combination according to any one of the preceding embodiments, wherein said antibody specific for CD19 and bendamustine are administered separately.

7. A combination according to any one of the preceding embodiments, wherein bendamustine is administered prior to administration of the antibody specific for CD19.

8. A combination according to any one of the preceding embodiments, which is able to mediate killing of MEC-1 cells by ADCC in the presence of isolated human PBMCs with an at least two-fold better efficacy than bendamustine alone.

9. A combination according to any one of the preceding embodiments for use in the treatment of non-Hodgkin's lymphoma, wherein the non-Hodgkin's lymphoma is selected from the group consisting of follicular lymphoma, small lymphocytic lymphoma, mucosa-associated lymphoid tissue, marginal zone, diffuse large B cell, Burkitt's, and mantle cell.

## Claims

1. A combination of an antibody specific for CD19 and a nitrogen mustard for use in the treatment of non-Hodgkin's lymphoma, chronic lymphocytic leukemia or acute lymphoblastic leukemia, wherein the antibody comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6), and wherein the nitrogen mustard is cyclophosphamide, chlorambucil, uramustine, ifosfamide or melphalan.

2. An antibody specific for CD19 wherein said antibody comprises an HCDR1 region comprising sequence SYVMH (SEQ ID NO: 1), an HCDR2 region comprising sequence NPYNDG (SEQ ID NO: 2), an HCDR3 region comprising sequence GTYYYGTRVFDY (SEQ ID NO: 3), an LCDR1 region comprising sequence RSSKSLQNVNGNTYLY (SEQ ID NO: 4), an LCDR2 region comprising sequence RMSNLNS (SEQ ID NO: 5), and an LCDR3 region comprising sequence MQHLEYPIT (SEQ ID NO: 6) for use in combination with a nitrogen mustard in the treatment of non-Hodgkin's lymphoma, chronic lymphocytic leukemia and/or acute lymphoblastic leukemia, wherein the nitrogen mustard is cyclophosphamide, chlorambucil, uramustine, ifosfamide or melphalan.

3. A nitrogen mustard for use in combination with an antibody specific for CD19 wherein said antibody comprises an HCDR1 region comprising sequence SYVMH (SEQ ID NO: 1), an HCDR2 region comprising sequence NPYNDG (SEQ ID NO: 2), an HCDR3 region comprising sequence GTYYYGTRVFDY (SEQ ID NO: 3), an LCDR1 region comprising sequence RSSKSLQNVNGNTYLY (SEQ ID NO: 4), an LCDR2 region comprising sequence RMSNLNS (SEQ ID NO: 5), and an LCDR3 region comprising sequence MQHLEYPIT (SEQ ID NO: 6) in the treatment of non-Hodgkin's lymphoma, chronic lymphocytic leukemia and/or acute lymphoblastic leukemia, and wherein the nitrogen mustard is cyclophosphamide, chlorambucil, uramustine, ifosfamide or melphalan.

4. The combination for use according to any one of the preceding claims, wherein the antibody comprises a variable heavy chain of the sequence EVQLVESGGGLVKPGGSLKLSCAASGYTFTSYVMHWVRQAPGKGLEWIGYNPYNDG TKYNEKFQGRVTISSDKSISTAYMELSSLRSEDTAMYYCARGTYYYGTRVFDYWGQG TLVTVSS (SEQ ID NO: 10) and a variable light chain of the sequence

DIVMTQSPATLSLSPGERATLSCRSSKSLQNVNGNTYLYWFQQKPGQSPQLLIYRMSN LNSGVPDRFSGSGSGTEFTLTISSLEPEDFAVYYCMQHLEYPITFGAGTKLEIK (SEQ ID NO: 11).

5. The combination for use according to any one of the preceding claims, wherein the antibody comprises a heavy chain constant domain of the sequence

ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTC
PPCPAPELLGGPDVFLFPPKPKDTLMISRTPEVTCVVVDVSHEDPEVQFNWYVDGVEV
HNAKTKPREEQFNSTFRVVSVLTVVHQDWLNGKEYKCKVSNKALPAPEEKTISKTKGQ
PREPQVYTLPPSREEMTKNQVSLTCLVKGFYPSDIAVEWESNGQPENNYKTTPPMLD
SDGSFFLYSKLTVDKSRWQQGNVFSCSVMHEALHNHYTQKSLSLSPGK (SEQ ID NO: 12).

6. The combination for use according to any one of the preceding claims, wherein the antibody comprises a light chain constant domain of the sequence

RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ ID NO: 13).

7. The combination for use according to any one of the preceding claims, wherein the treatment is of non-Hodgkin's lymphoma, optionally wherein the non-Hodgkin's lymphoma is selected from the group consisting of follicular lymphoma, small lymphocytic lymphoma, mucosa-associated lymphoid tissue lymphoma, marginal zone lymphoma, diffuse large B cell lymphoma, Burkitt's lymphoma, and mantle cell lymphoma.

8. The combination for use according to any one of claims 1-6, wherein the treatment is of chronic lymphocytic leukemia.

9. The combination for use according to any one of claims 1-6, wherein the treatment is of acute lymphoblastic leukemia.

10. The combination for use according to any one of the preceding claims, wherein the antibody specific for CD19 and the nitrogen mustard are:

(a) formulated together in one pharmaceutical composition; or
(b) formulated in different pharmaceutical compositions.

11. The combination for use according to any one of claims 1 to 10, wherein the antibody specific for CD19 and the nitrogen mustard are administered simultaneously.

12. The combination for use according to any one of claims 1 to 9 or claim 10(b), wherein the antibody specific for CD19 and the nitrogen mustard are administered separately.

# Figure 1

## Cytotoxicity of MOR00208 and BEN alone and in combination

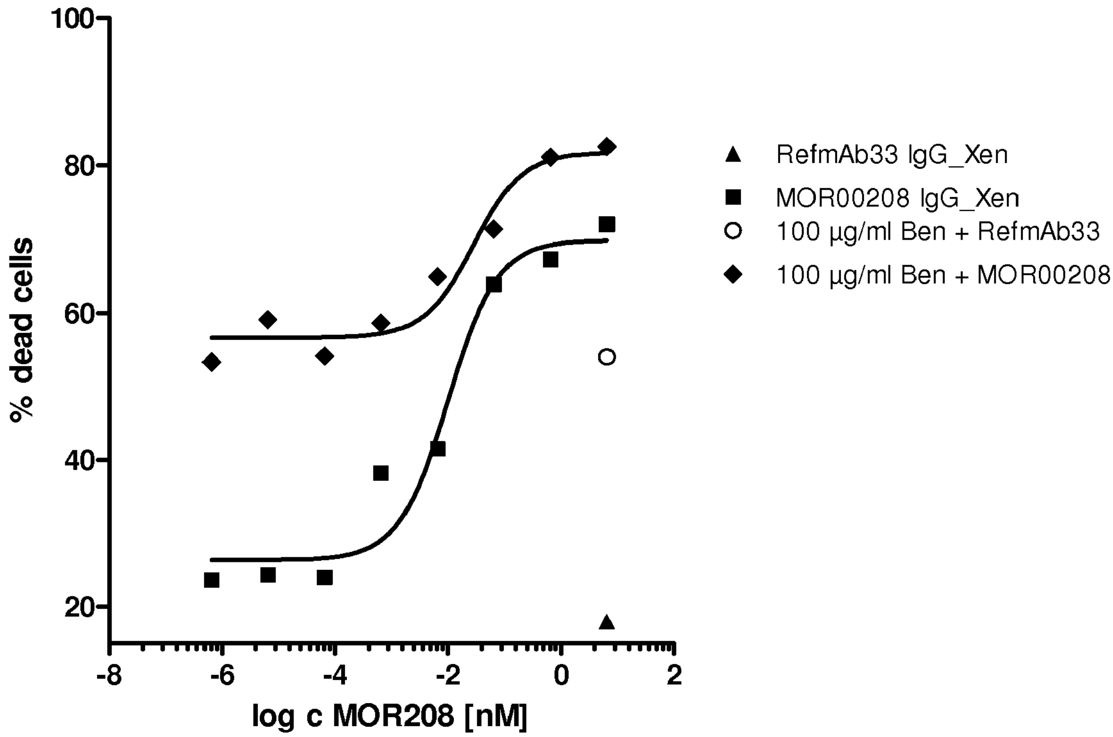

# Figure 2

## ADCC Dose response curves

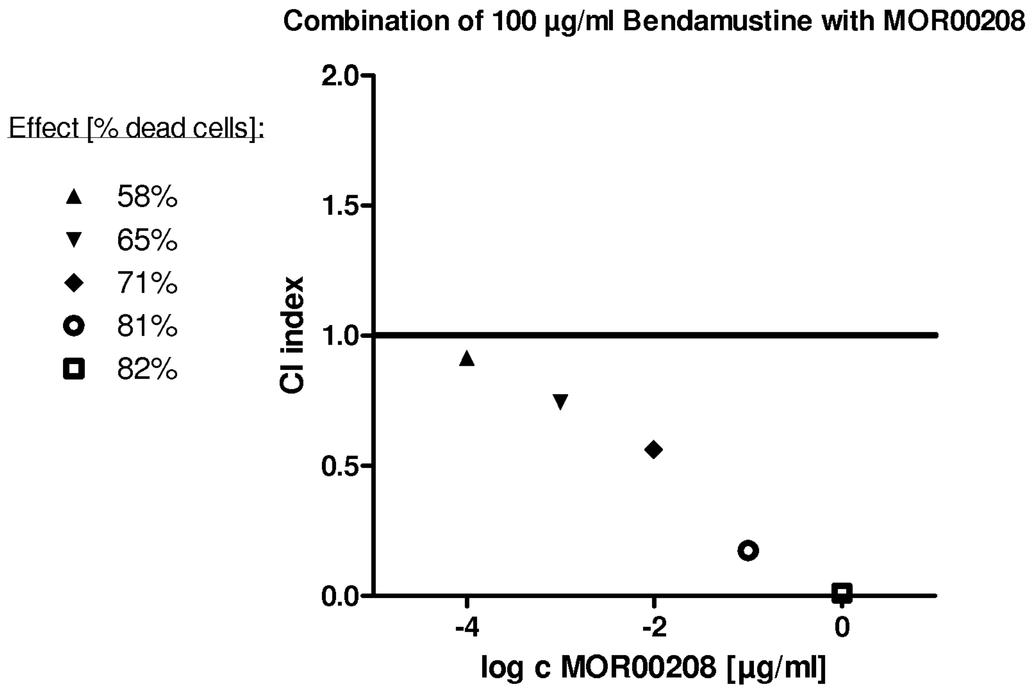

## Figure 3

The amino acid sequence of the MOR00208 Variable Heavy Domain is:
(The CDRs are bolded and underlined)

EVQLVESGGGLVKPGGSLKLSCAASGYTFT**<u>SYVMH</u>**WVRQAPGKGLEWIGYI**<u>NPYNDG</u>**TKYNEKFQGRVTISSDKSISTAYMELSSLRSEDTAMYYCAR**<u>GTYYYGTRVFDY</u>**WGQGTLVTVSS (SEQ ID NO: 10)

The amino acid sequence of the MOR00208 Variable Light Domain is:
(The CDRs are bolded and underlined)

DIVMTQSPATLSLSPGERATLSC**<u>RSSKSLQNVNGNTYLY</u>**WFQQKPGQSPQLLIY**<u>RMSNLNS</u>**GVPDRFSGSGSGTEFTLTISSLEPEDFAVYYC**<u>MQHLEYPIT</u>**FGAGTKLEIK (SEQ ID NO: 11)

The amino acid sequence of the MOR00208 HCDR1 is: SYVMH (SEQ ID NO: 1)

The amino acid sequence of the MOR00208 HCDR2 is: NPYNDG (SEQ ID NO: 2)

The amino acid sequence of the MOR00208 HCDR3 is: GTYYYGTRVFDY (SEQ ID NO: 3)

The amino acid sequence of the MOR00208 LCDR1 is: RSSKSLQNVNGNTYLY (SEQ ID NO: 4)

The amino acid sequence of the MOR00208 LCDR2 is: RMSNLNS (SEQ ID NO: 5)

The amino acid sequence of the MOR00208 LCDR3 is: MQHLEYPIT (SEQ ID NO: 6)

# Figure 4

## Sequence of Fc regions

The amino acids sequence of the MOR00208 heavy chain Fc region is:

ASTKGPSVFPLAPSSKSTSGGTAALGCLVKD YFPEPVTVSWNSGALTSGVH TFPAVLQSSGLYSLSSWTVPSSSLGTQTYICNVNHKPSNTKVDKKVEPKSCDKTHTCPPC PAPELLGGPDVFLFPPKPKDTLMISRTPEVTCVVVDVSHEDPEVQFNWYVDGVEVHNAKT KPREEQFNSTFRVVSVLTVVHQDWLNGKEYKCKVSNKALPAPEEKTISKTKGQPREPQV YTLPPSREEMTKNQVSLTCLVKGFYPSDIAVEWESNGQPENNYKTTPPMLDSDGSFFLYS KLTVDKSRWQQGNVFSCSVMHEALHNHYTQKSLSLSPGK (SEQ ID NO: 12)

The amino acids sequence of the MOR00208 light chain Fc region is:

RTVAAPSVFIFPPSDEQLKSGTASVVCLLNNFYPREAKVQWKVDNALQSGNSQESVTEQ DSKD STYSLSSTLTLSKADYEKHKVYACEVTHQGLSSPVTKSFNRGEC (SEQ ID NO: 13)

# Figure 5

Normalized specific killing; SD; MEC-1 target cells pretreated with Bendamustine (Ben) for 48 h before ADCC; pool of 3 independent experiments with 3 different effector cell donors

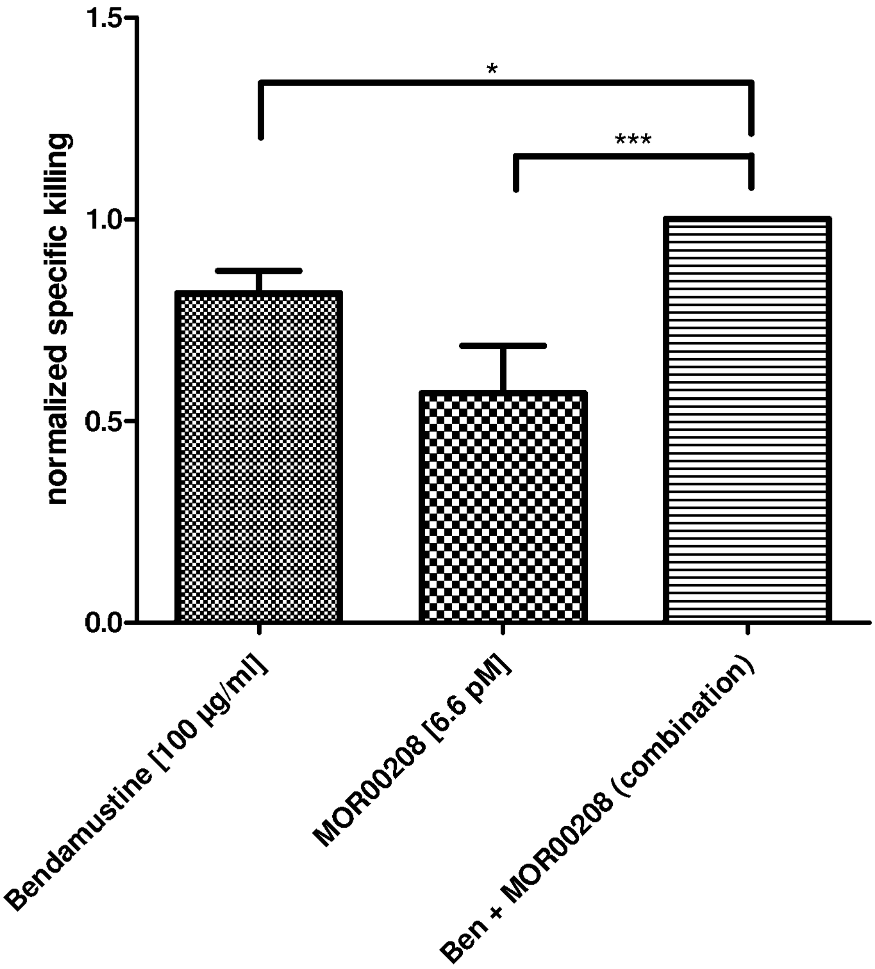


The figure shows the averages from the data shown in Table 2.

# Figure 6

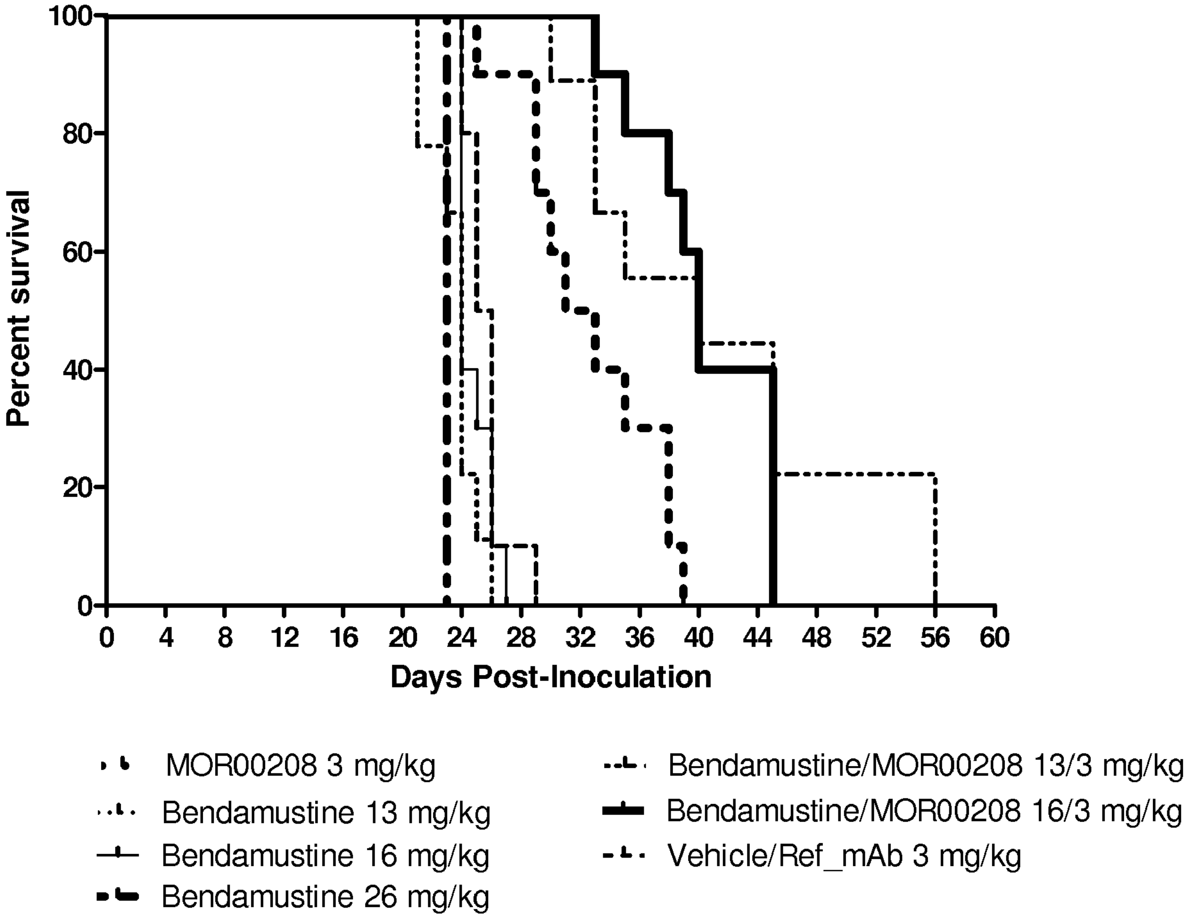

Percent survival
100
80
60
40
20
0
0
4
8
12
16
20
24
28
32
36
40
44
48
52
56
60
Days Post-Inoculation
MOR00208 3 mg/kg
Bendamustine 13 mg/kg
Bendamustine 16 mg/kg
Bendamustine 26 mg/kg
Bendamustine/MOR00208 13/3 mg/kg
Bendamustine/MOR00208 16/3 mg/kg
Vehicle/Ref_mAb 3 mg/kg

## Figure 7

| | Log rank test [Comparison of Kaplan Meyer curves] | One-Way-ANOVA + Bonferroni Multiple Comparison Test |
|---|---|---|
| vehicle control vs MOR00208 [6 or 10 mg/kg] | ns | ns |
| vehicle control vs Bendamustine [13 mg/kg] | ns | ns |
| vehicle control vs Bendamustine [16 mg/kg] | ns | ns |
| vehicle control vs MOR00208/Bendamustine [10/13 mg/kg] | ns | ns |
| vehicle control vs MOR00208/Bendamustine [6 or 10/16 mg/kg] | * p = 0.048 | ns |
| MOR00208 10 mg/kg vs MOR208/Bendamustine [6 or 10/16 mg/kg] | ns | ns |
| MOR00208 10 mg/kg vs MOR208/Bendamustine [6 or 10/13 mg/kg] | ns | ns |
| Bendamustine 16 mg/kg vs MOR208/Bendamustine [6 or 10/16 mg/kg] | ns | ns |
| Bendamustine 13 mg/kg vs MOR208/Bendamustine [6 or 10/13 mg/kg] | * p = 0.0266 | ns |

# Figure 8

| | Median days to 4000 mg | Increased time to 4000 mg [%] | Mean Tumor size at study day 34 [mg] | Reduced tumor size at study day 34 [%] |
|---|---|---|---|---|
| Vehicle control/Ref_mAb_33 | 38.1 | 0.00 | 2683 | 0.0 |
| MOR00208 [6 or 10 mg/kg] | 39.7 | 4.20 | 2375 | 11.5 |
| Bendamustine [13 mg/kg] | 38.3 | 0.52 | 2593 | 3.4 |
| Bendamustine [16 mg/kg] | 40.8 | 7.09 | 1948 | 27.4 |
| MOR208/Bendamustine [6 or 10/13 mg/kg] | 42.2 | 10.76 | 2261 | 15.7 |
| MOR208/Bendamustine [6 or 10/16 mg/kg] | 42.5 | 11.55 | 1771 | 34.0 |

# Figure 9

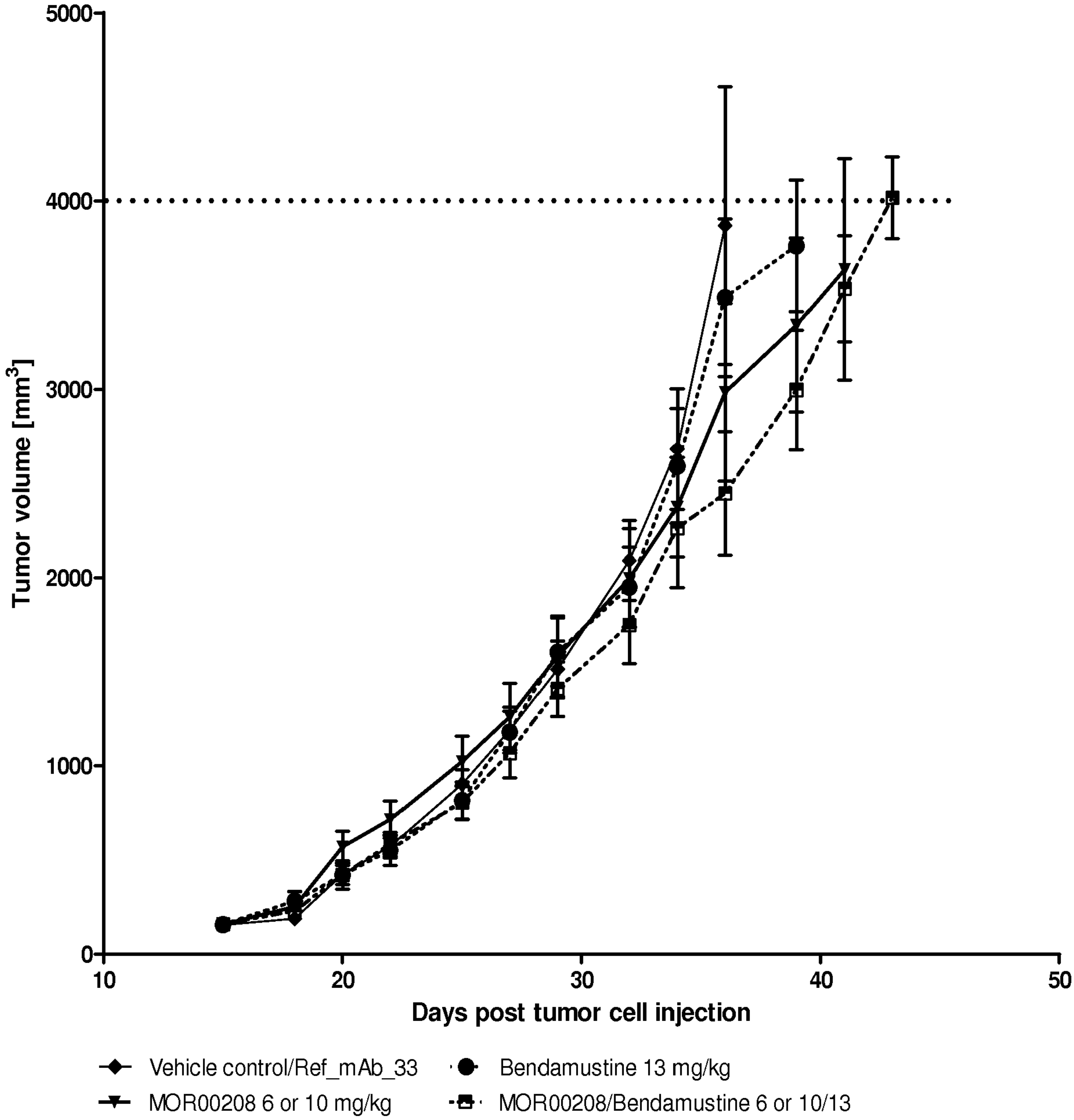
5000
4000
3000
2000
1000
0
Tumor volume [mm3]
10
20
30
40
50
Days post tumor cell injection
Vehicle control/Ref_mAb_33
Bendamustine 13 mg/kg
MOR00208 6 or 10 mg/kg
MOR00208/Bendamustine 6 or 10/13

# Figure 10

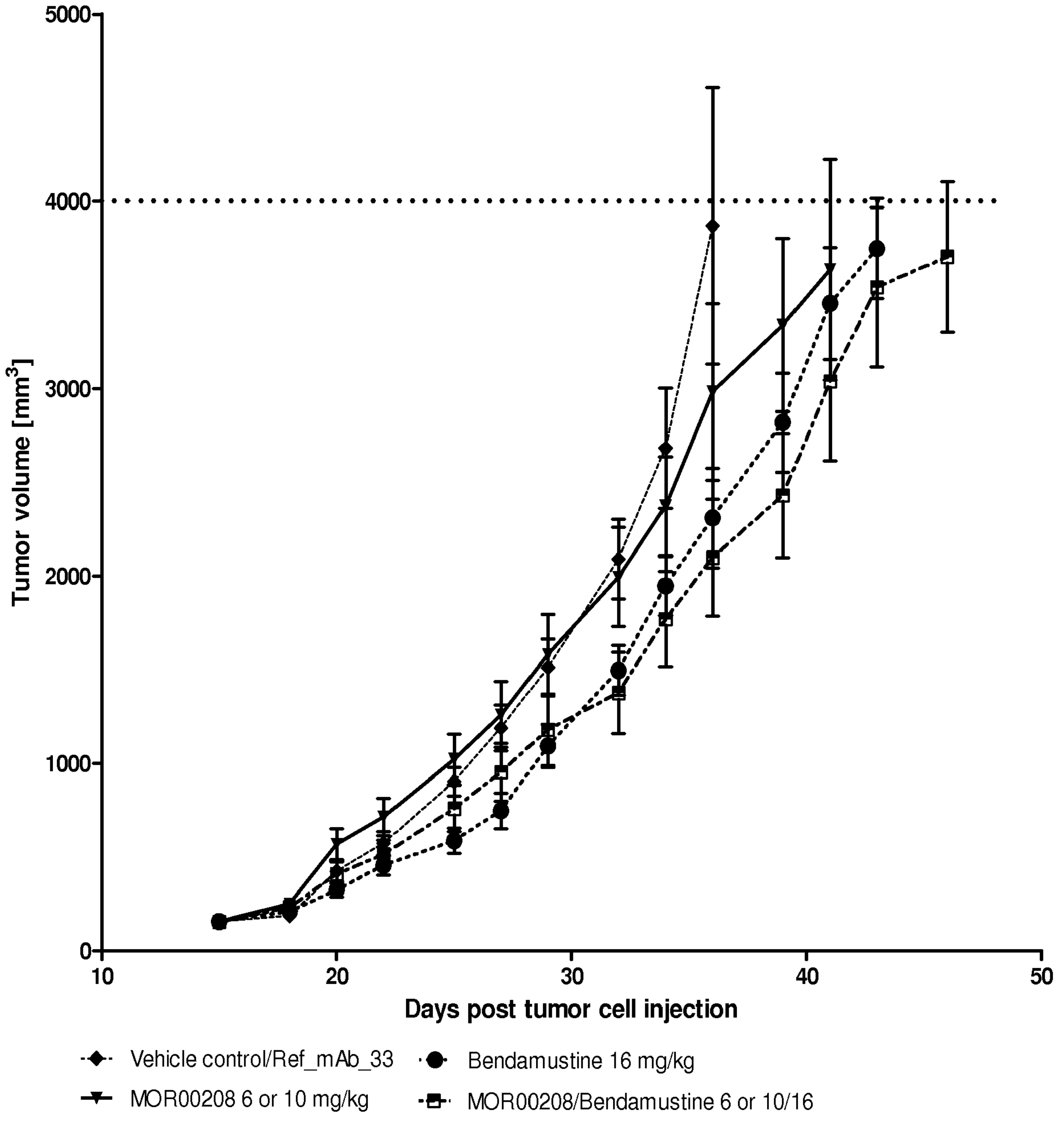
5000
4000
3000
2000
1000
0
10
20
30
40
50
Tumor volume [mm3]
Days post tumor cell injection
Vehicle control/Ref_mAb_33
Bendamustine 16 mg/kg
MOR00208 6 or 10 mg/kg
MOR00208/Bendamustine 6 or 10/16

Europäisches Patentamt
European Patent Office
Office européen des brevets

# EUROPEAN SEARCH REPORT

Application Number
EP 22 15 5572

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2007/154473 A1 (SUPER MICHAEL [US] ET AL) 5 July 2007 (2007-07-05)<br>* paragraph [0151] *<br>----- | 1-12 | INV.<br>A61K39/395<br>C07K16/28<br>A61K31/4184<br>A61P35/00<br>A61P35/02<br>A61P43/00<br>C07K16/30 |
| X,D | WO 2007/076950 A1 (MERCK PATENT GMBH [DE]; SUPER MICHAEL [US]; DAVIS JONATHAN H [US]; STE) 12 July 2007 (2007-07-12)<br>* page 41 *<br>----- | 1-12 | |
| A,D | KALOS MICHAEL ET AL: "T Cells with Chimeric Antigen Receptors Have Potent Antitumor Effects and Can Establish Memory in Patients with Advanced Leukemia",<br>SCIENCE TRANSLATIONAL MEDICINE,<br>vol. 3, no. 95,<br>10 August 2011 (2011-08-10), XP002667262,<br>* the whole document *<br>----- | 1-12 | |
| A,D | WO 2006/065392 A2 (CEPHALON INC [US]; BENDALL HEATHER HELENE [US]; ELLIOTT GARY T [US]; L) 22 June 2006 (2006-06-22)<br>* the whole document *<br>----- | 1-12 | TECHNICAL FIELDS SEARCHED (IPC) |
| A,D | BREMER KARL: "High rates of long-lasting remissions after 5-day bendamustine chemotherapy cycles in pre-treated low-grade non-Hodgkin's-lymphomas",<br>JOURNAL OF CANCER RESEARCH AND CLINICAL ONCOLOGY, SPRINGER INTERNATIONAL, BERLIN, DE,<br>vol. 128, no. 11,<br>1 November 2002 (2002-11-01), pages 603-609, XP002551065,<br>ISSN: 0171-5216, DOI: 10.1007/S00432-002-0378-6<br>[retrieved on 2002-10-26]<br>* the whole document *<br>-----<br>-/-- | 1-12 | A61K<br>C07K |

The present search report has been drawn up for all claims

1

| Place of search | Date of completion of the search | Examiner |
|---|---|---|
| Munich | 1 August 2022 | Irion, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 5572

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | SCHEUERMANN R H ET AL: "CD19 antigen in leukemia and lymphoma diagnosis and immunotherapy", LEUKEMIA AND LYMPHOMA, HARWOOD ACADEMIC PUBLISHERS, CHUR, CH, vol. 18, no. 5-6, 1 August 1995 (1995-08-01), pages 385-397, XP009155469, ISSN: 1042-8194 * the whole document * ----- | 1-12 | |
| X,D | SADELAIN MICHEL ET AL: "The promise and potential pitfalls of chimeric antigen receptors", CURRENT OPINION IN IMMUNOLOGY, ELSEVIER, OXFORD, GB, vol. 21, no. 2, 2 April 2009 (2009-04-02), pages 215-223, XP002667114, ISSN: 0952-7915 * the whole document * ----- | 1-12 | |
| X,D | WO 2008/150494 A1 (XENCOR INC [US]; CHU SEUNG YUP [US]; DESJARLAIS JOHN R [US]; KARKI SHE) 11 December 2008 (2008-12-11) * page 77 * ----- | 1-12 | TECHNICAL FIELDS SEARCHED (IPC) |

The present search report has been drawn up for all claims

1

| Place of search | Date of completion of the search | Examiner |
|---|---|---|
| Munich | 1 August 2022 | Irion, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 22 15 5572

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2022

| Patent document cited in search report | Publication date | Patent family member(s) | Publication date |
|---|---|---|---|
| US 2007154473 A1 | 05-07-2007 | AT 509954 T | 15-06-2011 |
| | | AU 2006332155 A1 | 12-07-2007 |
| | | BR PI0620795 A2 | 22-11-2011 |
| | | CA 2635623 A1 | 12-07-2007 |
| | | CN 101351477 A | 21-01-2009 |
| | | DK 1966245 T3 | 18-07-2011 |
| | | DK 2270050 T3 | 12-08-2013 |
| | | EA 200870132 A1 | 30-12-2008 |
| | | EP 1966245 A1 | 10-09-2008 |
| | | EP 2270050 A1 | 05-01-2011 |
| | | ES 2365046 T3 | 21-09-2011 |
| | | ES 2426468 T3 | 23-10-2013 |
| | | JP 6104332 B2 | 29-03-2017 |
| | | JP 2009521912 A | 11-06-2009 |
| | | JP 2016014039 A | 28-01-2016 |
| | | KR 20080090485 A | 08-10-2008 |
| | | PT 1966245 E | 31-08-2011 |
| | | PT 2270050 E | 12-09-2013 |
| | | US 2007154473 A1 | 05-07-2007 |
| | | US 2014288288 A1 | 25-09-2014 |
| | | US 2015322159 A1 | 12-11-2015 |
| | | US 2019241671 A1 | 08-08-2019 |
| | | WO 2007076950 A1 | 12-07-2007 |
| | | ZA 200806609 B | 25-11-2009 |
| WO 2007076950 A1 | 12-07-2007 | AT 509954 T | 15-06-2011 |
| | | AU 2006332155 A1 | 12-07-2007 |
| | | BR PI0620795 A2 | 22-11-2011 |
| | | CA 2635623 A1 | 12-07-2007 |
| | | CN 101351477 A | 21-01-2009 |
| | | DK 1966245 T3 | 18-07-2011 |
| | | DK 2270050 T3 | 12-08-2013 |
| | | EA 200870132 A1 | 30-12-2008 |
| | | EP 1966245 A1 | 10-09-2008 |
| | | EP 2270050 A1 | 05-01-2011 |
| | | ES 2365046 T3 | 21-09-2011 |
| | | ES 2426468 T3 | 23-10-2013 |
| | | JP 6104332 B2 | 29-03-2017 |
| | | JP 2009521912 A | 11-06-2009 |
| | | JP 2016014039 A | 28-01-2016 |
| | | KR 20080090485 A | 08-10-2008 |
| | | PT 1966245 E | 31-08-2011 |
| | | PT 2270050 E | 12-09-2013 |
| | | US 2007154473 A1 | 05-07-2007 |
| | | US 2014288288 A1 | 25-09-2014 |
| | | US 2015322159 A1 | 12-11-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 22 15 5572

This annex lists the patent family members relating to the patent documents cited in the above-mentioned European search report. The members are as contained in the European Patent Office EDP file on The European Patent Office is in no way liable for these particulars which are merely given for the purpose of information.

01-08-2022

| Patent document cited in search report | Publication date | Patent family member(s) | Publication date |
|---|---|---|---|
| | | US 2019241671 A1 | 08-08-2019 |
| | | WO 2007076950 A1 | 12-07-2007 |
| | | ZA 200806609 B | 25-11-2009 |
| WO 2006065392 A2 | 22-06-2006 | AR 054094 A1 | 06-06-2007 |
| | | AU 2005317047 A1 | 22-06-2006 |
| | | CA 2585659 A1 | 22-06-2006 |
| | | CL 2009001721 A1 | 19-02-2010 |
| | | CN 101933923 A | 05-01-2011 |
| | | EP 1814544 A2 | 08-08-2007 |
| | | JP 2008519047 A | 05-06-2008 |
| | | TW 200621240 A | 01-07-2006 |
| | | US 2006128777 A1 | 15-06-2006 |
| | | US 2009209606 A1 | 20-08-2009 |
| | | WO 2006065392 A2 | 22-06-2006 |
| WO 2008150494 A1 | 11-12-2008 | AU 2008260498 A1 | 11-12-2008 |
| | | CA 2693053 A1 | 11-12-2008 |
| | | DK 2176298 T3 | 12-02-2018 |
| | | EP 2176298 A1 | 21-04-2010 |
| | | EP 2708557 A1 | 19-03-2014 |
| | | EP 3392273 A1 | 24-10-2018 |
| | | ES 2659517 T3 | 16-03-2018 |
| | | IL 202406 A | 29-12-2016 |
| | | IL 224397 A | 31-05-2016 |
| | | LT 2176298 T | 10-04-2018 |
| | | US 2009136485 A1 | 28-05-2009 |
| | | US 2012148578 A1 | 14-06-2012 |
| | | US 2012156207 A1 | 21-06-2012 |
| | | US 2012156220 A1 | 21-06-2012 |
| | | US 2012321620 A1 | 20-12-2012 |
| | | US 2013315910 A1 | 28-11-2013 |
| | | US 2015037321 A1 | 05-02-2015 |
| | | US 2015037322 A1 | 05-02-2015 |
| | | US 2016122435 A1 | 05-05-2016 |
| | | US 2018215821 A1 | 02-08-2018 |
| | | US 2018215822 A1 | 02-08-2018 |
| | | US 2019315861 A1 | 17-10-2019 |
| | | US 2019330342 A1 | 31-10-2019 |
| | | US 2020071402 A1 | 05-03-2020 |
| | | US 2021009689 A1 | 14-01-2021 |
| | | US 2021009690 A1 | 14-01-2021 |
| | | US 2021395363 A1 | 23-12-2021 |
| | | WO 2008150494 A1 | 11-12-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61654097
- US 61647539
- US 61523861
- WO 2006065392 A
- WO 2007076950 A
- US 2007154473 A
- WO 2005012493 A
- US 7109304 B
- WO 2010053716 A
- US 12266999 B
- WO 2007002223 A
- US 8097703 B
- WO 2008022152 A
- WO 12377251 A
- WO 2008150494 A
- WO 2008031056 A
- US 11852106 B
- US 648505 B
- WO 2009052431 A
- US 12253895 B
- WO 2010095031 A
- WO 12710442 A
- WO 2010151341 A
- US 13377514 B
- US 5686072 A
- WO 2002022212 A
- US 0129026 W
- US 377251
- US 917750
- US 852106
- US 648505
- US 7968687 B
- US 710442
- WO 200348731 A
- US 4708871 A


### Non-patent literature cited in the description


- **NADLER et al.** *J. Immunol.,* 1983, vol. 131, 244-250
- **LOKEN et al.** *Blood,* 1987, vol. 70, 1316-1324
- **UCKUN et al.** *Blood,* 1988, vol. 71, 13-29
- **ANDERSON et al.** *Blood,* 1984, vol. 63, 1424-1433
- **SCHEUERMANN.** *Leuk. Lymphoma,* 1995, vol. 18, 385-397
- **GROSSBARD et al.** *Br. J. Haematol,* 1998, vol. 102, 509-15
- **TREON et al.** *Semin. Oncol,* 2003, vol. 30, 248-52
- **KALOS et al.** T cells with Chimeric Antigen Receptors Have Potent Antitumor Effects and Can Establish Memory in Patients with Advanced Leukemia. *Science Translational Medicine,* 10 August 2011, vol. 3 (95
- The promise and potential pitfalls of chimeric antigen receptors. **SADELAIN et al.** Current Opinion in Immunology. Elsevier, 02 April 2009, vol. 21
- High rates of long lasting remission after 5-day bendamustine chemotherapy cycles in pre-treated low-grade non-Hodgkin's lymphomas. **BREMER et al.** Journal of Cancer Research and Clinical Oncology. Springer International, 01 November 2002, vol. 128
- **SCHEUERMANN et al.** CD19 Antigen in Leukemia and Lymphoma Diagnosis and Immunotherapy. *Leukemia and Lymphoma,* 1995, vol. 18, 385-397
- **TING-CHAO CHOU.** Theoretical Basis, Experimental Design, and Computerized Simulation of Synergism and Antagonism in Drug Combination Studies. *Pharmacol Rev,* 2006, vol. 58, 621-681
- **CLARKE et al.** Issues in experimental design and endpoint analysis in the study of experimental cytotoxic agents in vivo in breast cancer and other models. *Breast Cancer Research and Treatment,* 1997, vol. 46, 255-278
- **WEBB, J. L.** Enzyme and Metabolic Inhibitors. Academic Press, 1963
- **CHOTHIA C ; LESK AM.** Canonical structures for the hypervariable regions of immunoglobulins. *J Mol Biol.,* 1987, vol. 196 (4), 901-17
- **KABAT E.A ; WU T.T. ; PERRY H.M. ; GOTTESMAN K.S. ; FOELLER C.** Sequences of Proteins of Immunological Interest. US Dept. of Health and Human Services, 1991
- Epitope Mapping Protocols in Methods in Molecular Biology. Humana Press, 1996, vol. 66
- **GEYSEN et al.** *Proc. Natl. Acad. Sci. USA,* 1984, vol. 8, 3998-4002

- **GEYSEN et al.** *Proc. Natl. Acad. Sci. USA,* 1985, vol. 82, 78-182
- **GEYSEN et al.** *Mol. Immunol.,* 1986, vol. 23, 709-715
- **HOPP et al.** *Proc. Natl. Acad. Sci USA,* 1981, vol. 78, 3824-3828
- **KYTE et al.** *J.Mol. Biol.,* 1982, vol. 157, 105-132
- **CHOU TC ; TALALAY P.** Quantitative analysis of dose-effect relationships: the combined effects of multiple drugs or enzyme inhibitors. *Adv Enzyme Regul,* 1984, vol. 22, 27-55
- **JAMES et al.** Antibody-mediated B-cell depletion before adoptive immunotherapy with T cells expressing CD20-specific chimeric T-cell receptors facilitates eradication of leukemia in immunocompetent mice. *Blood,* 30 October 2009, vol. 114 (27), 5454-63